(19) Europäisches Patentamt
European Patent Office
Office européen des brevets

(11) Publication number: 0 360 276 A2

(12) # EUROPEAN PATENT APPLICATION

(21) Application number: 89117516.8

(22) Date of filing: 22.09.89

(51) Int. Cl.5: C12Q 1/02 , C12N 11/08 , C08F 26/06 , //G01N33/18, C12Q1/54

(30) Priority: 22.09.88 JP 238187/88
22.09.88 JP 238188/88
28.09.88 JP 243529/88
26.04.89 JP 106908/89

(43) Date of publication of application: 28.03.90 Bulletin 90/13

(84) Designated Contracting States: DE ES FR GB IT

(71) Applicant: KAO CORPORATION
14-10, Nihonbashi Kayaba-cho 1-chome
Chuo-ku Tokyo(JP)

(72) Inventor: Sakata, Masaru
283-24, Hatake Iwadecho
Naga-gun Wakayama(JP)
Inventor: Tanigaki, Masanobu
845-9, Otani
Wakayama-shi Wakayama(JP)
Inventor: Kondo, Akihiro
8-59, Nishihama 3-chome
Wakayama-shi Wakayama(JP)
Inventor: Totoki, Shintaro
1130, Nishihama
Wakayama-shi Wakayama(JP)
Inventor: Kawabata, Nariyoshi
2-24, Shioji 1-chome Nishinari-ku
Osaka-shi Osaka(JP)
Inventor: Mimura, Koji
117, Nakaguro Iwadecho
Naga-gun Wakayama(JP)
Inventor: Takaya, Hitoshi
1130, Nishihama
Wakayama-shi Wakayama(JP)
Inventor: Konishi, Noriko
1430, Kada
Wakayama-shi Wakayama(JP)

(74) Representative: Hansen, Bernd, Dr.rer.nat. et al
Hoffmann, Eitle & Partner Patentanwälte
Arabellastrasse 4 Postfach 81 04 20
D-8000 München 81(DE)

(54) **Microbial adsorbent and microbial sensor using the same.**

(57) A microbial sensor, which comprises a container packed with a microbial adsorbent on which an aerobic microorganism is immobilized and an electrochemical device whereby a substance consumed or produced under the effects of said microorganism is determined, is disclosed. The microbial sensor of the present invention makes it possible to rapidly and readily determine, for example, BOD at a high accuracy.

EP 0 360 276 A2

# MICROBIAL ADSORBENT AND MICROBIAL SENSOR USING THE SAME

## FIELD OF THE INVENTION

This invention relates to a microbial adsorbent and a microbial sensor using the same.

## BACKGROUND OF THE INVENTION

Recently the application range of sensors has been more and more enlarged and various sensors have been developed. Sensors may be roughly classified into two types, namely, physical sensors which are employed in measuring, for example, temperature, moisture and pressure and chemical ones which are employed in the determination of various chemicals. Among the chemical sensors, those wherein a microorganism is used in a molecule-distinguishing element are generally called microbial sensors.

It has been known for a long time that a microorganism would adhere to the surface of a polymer and thus a strongly basic anion exchange resin has been employed in order to collect microorganisms in water. Furthermore, bioreactors wherein microorganisms are adsorbed by an ion exchange derivative of a polysaccharide or an ion exchange resin have been widely applied.

It has been attempted to determine BOD (biochemical oxygen demand) within a short period of time by using a microbial sensor as described, for example, in Biotechnology and Bioengineering, vol. XIX, pages 1535 - 1547 (1977).

This sensor comprises a specific microorganism included in a porous acetyl cellulose membrane which is bonded to the surface of a membrane oxygen electrode. However, the application of this microbial sensor is limited to specific microorganisms. Furthermore, the BOD value determined by using it would commonly be in disagreement with one determined by a standard method for determining BOD, for example, the one specified in JIS K0102 (the term "JIS" as used herein means Japanese Industrial Standard"). Accordingly, it is required that one determine an equation showing the relationship between these two values so as to make them to agree with each other. These problems make this microbial sensor less useful in practice.

As a result of extensive studies for a microbial sensor which would enable one to obtain a rapid and accurate determination of the concentration of a substance relating to the consumption of oxygen, in particular, BOD, we have previously developed a microbial sensor comprising a water insoluble polymer on which an aerobic microorganism is adsorbed together with a membrane oxygen electrode, and have applied for a patent on the same, i.e., JP-A-62-238454 (the term "JP-A" as used herein means an "unexamined published Japanese Patent Application").

The microbial sensor according to the JP-A-62-238454 comprises an aerobic microorganism adsorbed by a water insoluble polymer in which comprises a matrix selected from among vinyl polymers (e.g., crosslinking polystyrene and crosslinking polyvinyl pyridine), acrylic polymers (e.g., crosslinking polyacrylate or crosslinking polymethacrylate), condensed polymers (e.g., phenol/formaldehyde resin), and having an amino, a pyridyl, a tetraalkylammonium, a tetraallylammonium, a tetraarylammonium or a pyridinium group, is located below a membrane oxygen electrode. The microbial sensor disclosed in JP-A-62-238454 is prepared, for example, by the method in which a rubber stopper 4, which has a pore size almost the same as that of a membrane oxygen electrode 1, is partly recessed into the bottom of the electrode 1 to thereby give a space surrounded by the inner wall of the rubber stopper 4 and the bottom of the oxygen electrode 1. Then this space is charged with a water insoluble polymer 3 and the bottom opening of the rubber stopper 4 is covered with a net 5 to thereby carry and fix said polymer 3, as shown in Fig. 1.

The application of this sensor to the determination of BOD will be illustrated by way of example. First, a part packed with the resin is immersed in a microbial suspension to thereby allow the resin to adsorb the microorganism. Next, the sensor is washed with, for example, a buffer solution or deionized water. When the dissolved oxygen concentration is stabilized, an organic material is added thereto. Then the dissolved oxygen concentration slowly decreases and becomes constant within 20 to 30 minutes. It has been confirmed that the decrease in the dissolved oxygen concentration correlate to the concentration of the organic material. Thus the BOD can be determined by measuring the decrease in the dissolved oxygen concentration.

The abovementioned microbial sensor is not completely free from drawbacks. In the structure shown in

Fig. 1, the solution migrates from the part packed with the resin to the electrode mostly through diffusion. The migration rate of the solution in the part packed with the resin is extremely low. Therefore the stabilization of the dissolved oxygen concentration indicated by the electrode requires a considerably long period of time. Furthermore, it is sometimes observed that the dissolved oxygen concentration does not return to the base line after the measurement, for example, when the concentration of the sample is high.

BOD has been generally determined by a method specified in JIS K0102. However, this method requires a determination period as long as five days and a complicated procedure. Thus it should be conducted by a skilled person.

In the analysis of BOD by using a microbial sensor, it is therefore ideal to minimize the determination period by minimizing the load of a microbial adsorbent and elevating the flow rate of a buffer solution so as to allow the indication of oxygen concentration to return to the base line value within a short period of time.

In order to form such an ideal sensor system as described above, it is desirable to adsorb and immobilize as much of a vital microorganism as possible on a water insoluble carrier. It is preferable to adsorb and immobilize at least 0.1 mg, still preferably at least 1 mg, of such a microorganism in terms of dry matter per gram of a carrier.

On the other hand, recently it has been reported that when a water insoluble polymer having a pyridinium group, wherein a 4-vinylpyridinium halide having a benzyl group, an alkyl group having 4 to 16 carbon atoms and a pentafluorophenylmethyl group, each as a substituent on a nitrogen atom, is vinyl-polymerized with a divinyl compound, the same is useful as a microbial adsorbent (see JP-B-62-41641 (the term "JP-B" as used herein means an "examined Japanese Patent Publication")).

It is considered that the abovementioned resin disclosed by JP-B-62-41641 is an excellent microbial adsorbent. However, the vinyl polymerization results in the formation of a crosslinking resin, which makes it difficult to obtain a fibrous or filmy resin, though a spherical, bulk or powdery one can be readily obtained thereby. In addition, the spherical, bulk or powdery resin thus obtained is disadvantageous from an economical point of view, since not the inside of the same but only its surface is available for the adsorption of microorganisms. When the vinyl pyridine crosslinking polymer is to be quaternized so as to give the aimed resin, it is sometimes observed that the insufficient quaternization of the inside of the resin would cause the formation of a salt from the unreacted vinyl pyridine moiety with an acid derived from a quaternizing agent such as a hydrogen halide. The use of such a resin in an aqueous system causes a problem that the pH value of the aqueous solution may vary with the lapse of time.

Therefore it has been required to provide a microbial adsorbent free from any of these problems in the prior art.

## SUMMARY OF THE INVENTION

Under these circumstances, we have conducted extensive studies in order to improve the sensitivity of the abovementioned microbial sensors. As a result, we have found that a microbial sensor comprising a specific structure is extremely effective for the determining, for example, of BOD, and further, a microbial adsorbent comprising a specific water insoluble polymer has a high absorbability for an aerobic microorganism and thus makes it possible to prepare a microbial sensor of an extremely high sensitivity, thus completing the requirements of the present invention.

Accordingly, the present invention provides a microbial sensor comprising a microbial adsorbent in which an aerobic microorganism is adsorbed, wherein the microbial sensor comprises:

a container packed with said microbial adsorbent selected from

(a) a polymer whose surface is coated with a polymer containing in the molecule thereof a pyridinium group represented by formula (I):

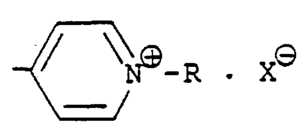

(I)

wherein R represents a benzyl group, a phenethyl group, an alkyl group having from 1 to 16 carbon atoms, or a pentafluorophenyl methyl group, and X represents a halogen atom; or

(b) a weakly basic anion exchange resin; and an electrochemical device which can determine a material formed or consumed under the effects of the microorganism, said container and said electrochemical device being separately provided in said microbial sensor.

The present invention further provides a microbial adsorbent comprising a molded product of a water insoluble polymer whose surface is coated with a substituted polyvinylpyridinium polymer which is substituted by a group(s) selected from the group consisting of a benzyl group, a phenethyl group, an alkyl group having 1 to 16 carbon atoms and a pentafluorophenylmethyl group as substituent(s) on a nitrogen atom of the substituted polyvinylpyridinium.

## BRIEF DESCRIPTION OF THE DRAWINGS

Fig. 1 shows a microbial sensor of JP-A-62-238454.

Fig. 2 shows an example of an embodiment of the microbial sensor of the present invention.

Fig. 3 shows changes in the dissolved oxygen concentrations of samples showing various BOD values determined by the microbial sensor of the present invention shown in Fig. 2.

Fig. 4 shows a relationship between the reciprocal of dissolved oxygen concentration determined by the microbial sensor of the present invention and the reciprocal of the BOD of a sample.

Fig. 5 shows a relationship between the reciprocal of the decreasing rate of the dissolved oxygen concentration determined by the microbial sensor of the present invention and the reciprocal of the BOD of a sample.

Fig. 6 shows another example of an embodiment of the microbial sensor of the present invention.

Fig. 7 shows a relationship between the reciprocal of the dissolved oxygen concentration determined by the microbial sensor of Fig. 6 and the reciprocal of the BOD of a sample.

Fig. 8 shows a relationship between the reciprocal of the dissolved oxygen concentration determined by the microbial sensor of the present invention and the reciprocal of the concentration of glucose in a sample.

Fig. 9 shows another example of an embodiment of the microbial sensor of the present invention.

Fig. 10 shows a relationship between the reciprocal of the dissolved oxygen concentration determined by the microbial sensor of Fig. 9 before and after the microbial reaction and the reciprocal of the BOD of a sample.

Figs. 11 to 17 show each a relationship between the dissolved oxygen concentration and the reciprocal of the BOD of a sample determined by using various microbial adsorbents.

## DETAILED DESCRIPTION OF THE INVENTION

The polymer containing a pyridinium group of formula (I) (hereinafter referred to as a "substituted pyridinium polymer") of the microbial adsorbent (a) include a water insoluble polymer comprising, as a polymer unit thereof, a vinylpyridinium halide having a substituent selected from a benzyl group, a phenethyl group, an alkyl group having 1 to 16, preferably from 1 to 12 carbon atoms and pentafluorophenylmethyl groups on the nitrogen atom of the vinylpyridinium halide; a divinyl compound; and, if desired, a vinyl monomer which can be copolymerized with the vinylpyridininium halide or divinyl compound.

The microbial adsorbent, whose surface is coated with the abovementioned substituted pyridinium polymer, may exclusively comprise said substituted pyridinium polymer. Alternately, it may comprise a molded product of another water insoluble polymer whose surface is coated with the substituted pyridinium polymer.

A microbial adsorbent exclusively comprises a substituted polymer may be prepared by a known method, for example, by polymerizing vinylpyridine together with a divinyl compound and, if desired, a vinyl monomer(s) which can be copolymerized with the vinylpyridine and divinyl compound and then the nitrogen atom in the pyridine ring is quaternized with an alkylating agent.

Among them, crosslinking polymer beads which are obtained by suspension polymerizing a monomer mixture containing a vinylpyridine monomer and a crosslinkable monomer with the use of a slightly water-soluble inorganic salt as a dispersion stabilizer, and further quaternizing a pyridyl group in the polymer beads thus obtained, to give repeating unit represented by the following formula (II):

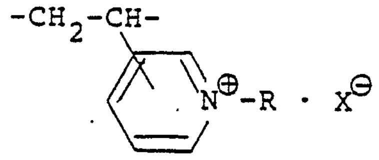

(II)

wherein R represents an alkyl group having from 1 to 16, preferably from 1 to 12 carbon atoms, a benzyl group, a phenethyl group, a phenylpropyl group or a pentafluorophenylmethyl group; and
$X^{\ominus}$ represents a chlorine or a bromine ion:
are particularly preferred from the viewpoint of microbial adsorbability.

The crosslinking polymer beads having the repeating unit represented by the formula (II) to be used in the present invention may be prepared by suspension polymerizing a vinylpyridine monomer with a crosslinkable monomer such as divinylbenzene, ethylene glycol di(meth)acrylate, tetramethylene glycol di(meth)acrylate or glycerol tri(meth)acrylate in oil-in-water type suspensions, and quaternizing the polymer beads thus obtained with the corresponding quaternizing agent.

A water soluble polymer such as gelatin, starch, hydroxyethyl cellulose, carboxymethyl cellulose or polyvinyl alcohol, or a slightly water soluble inorganic salt such as calcium carbonate, calcium phosphate or calcium sulfate is commonly employed in suspension polymerization as a dispersion stabilizer. However the former one (i.e., a water soluble polymer) intensely remains on the surface of the resulting water insoluble polymer, which inhibits the subsequent adsorption of microorganisms.

Examples of vinylpyridine monomers useful in the present invention include 2-vinylpyridine, 3-vinylpyridine, 4-vinylpyridine, 2-methyl-5-vinylpyridine, 2-ethyl-5-vinylpyridine, 3-methyl-5-vinylpyridine, 2,3-dimethyl-5-vinylpyridine and 2-methyl-3-ethyl-5-vinylpyridine. These compounds may be used either alone, or in a mixture of two or more, in the present invention. Furthermore, these vinylpyridines may be copolymerized with other vinyl monomers. Examples of these other vinyl monomers include aromatic monovinyl compounds such as styrene, vinyltoluene, vinylnaphthalene and chlorostyrene; and aliphatic monovinyl compounds such as methyl acrylate, methyl methacrylate, acrylonitrile, ethyl acrylate, butyl acrylate, ethyl methacrylate, N-t-butylacrylamide and N-t-butylmethacrylamide.

Among the monomers cited above, those obtained by using 4-vinylpyridine are particularly preferable from the viewpoints, for example, of microbial adsorbability and sensor sensitivity.

The pyridyl group in the polymer beads obtained by the suspension polymerization may be further quaternized with various known quaternizing agents. However, a quaternizing agent represented by the following formula (III):
R-X (III)
wherein R represents an alkyl group having from 1 to 16, preferably from 1 to 12 carbon atoms, a benzyl group, a phenylethyl group, a phenylpropyl group or a pentafluorophenylmethyl group; and
X represents a halogen atom;
is thought suitable for meeting the requirements of the present invention.

The quaternization may be conducted in a conventional manner and the conditions therefor are not particularly restricted.

A microbial adsorbent comprising another water insoluble polymer whose surface is coated with the substituted pyridinium polymer may be obtained by coating the surface of the polymer carrier, which has been preliminarily prepared, with a substituted pyridinium polymer.

Any water insoluble polymer can be used in the present invention for the carrier of the substituted pyridinium polymer. The water insoluble polymer may be used in various forms such as granules, a powder, a mass, fibers, a fabric, a film or a sponge-like form.

A microbial adsorbent comprising another water insoluble polymer whose surface is coated with a substituted pyridinium polymer can be prepared, for example, in the following manner.

A vinylpyridine homopolymer or a copolymer of vinylpyridine with other monomer(s) (each hereinafter referred to as a "vinylpyridine polymer") is carried on a molded product of a water insoluble polymer having a halomethyl group represented by the following general formula (IV):
$XCH_2$- (IV)
wherein X represents a halogen atom;
(hereinafter referred to as a "molded halomethyl polymer"), by the quaternization of the pyridine ring of the vinylpyridine polymer with the halomethyl group of the halomethyl polymer. Next, the nitrogen atom of the pyridine ring of the vinylpyridine polymer which is not quaternized in the above quaternization is further quaternized with an alkylating agent.

The molded halomethyl polymer may be prepared by a method, for example, selected from among the following ones depending on the form of final product and application conditions. The term "halomethyl group" as used herein involves alkyl and aryl groups substituted with a halomethyl group such as ω-monohalide alkyl groups.

(i) A monomer having a haloalkyl group such as chloromethylstyrene, 2-chloroethylvinyl ether, 2-chloroethyl (meth)acrylate, 2-bromoethyl (meth)acrylate or vinyl chloroacetate is homopolymerized, or copolymerized with monomer(s) copolymerizable therewith.

(ii) A monomer having a tertiary amino group such as dimethylaminoethyl (methacrylate, dimethylaminoethyl (meth)acrylamide or dimethylaminostyrene is homopolymerized, or copolymerized with monomers copolymerizable therewith. The polymer thus obtained is then reacted with a dihalide such as 1 , 3-dibromopropane, 1,4-dibromobutane or bischloromethylbenzene.

(iii) A homopolymer comprising an aromatic monomer (e.g., styrene, methylstyrene or vinylnaphthalene); a copolymer comprising one of the abovementioned polymers with a monomer copolymerizable therewith; or a polymer having an aromatic ring (e.g., a polyester having an aromatic ring, polyamide having an aromatic ring, polyurethane having an aromatic ring or a phenol resin), is chloromethylated by a known method.

(iv) A tertiary amino group is introduced into a polymer having an aromatic ring, as those cited in the above (iii), by a Mannich reaction. Next, the obtained product is reacted with such a dihalide as those cited in the above (ii).

(v) A polymer compound having a hydroxyl group such as a homopolymer of polyvinyl alcohol, denatured polyvinyl alcohol, cellulose, chitin, hydroxyethyl (meth)acrylate or hydroxypropyl (meth)acrylate, or a copolymer of one of these monomers together with other monomer(s) copolymerizable therewith is haloacetylated by using a known acylating agent such as chloroacetic chloride, bromoacetic chloride, chloroacetic anhydride, bormoacetic anhydride, monochloroacetic acid, monobromoacetic acid, a monochloroacetate or a monobromoacetate.

(vi) A polymer compound having a hydroxyl group as those cited in the above (v) is reacted with epichlorohydrin or epibromohydrin.

(vii) An acetal bond is formed between a polymer compound such as polyvinyl alcohol, denatured polyvinyl alcohol or vinylon and chloroacetaldehyde.

Examples of the vinylpyridine polymers useful in the present invention include homopolymers of 4-vinylpyridine, 3-vinylpyridine and 2-vinylpyridine, copolymers thereof with each other and copolymers thereof with monomers copolymerizable therewith such as styrene and styrene derivatives, (meth)acrylates, (meth)acrylamides and their derivatives and (meth)acrylonitrile. Among these materials, those containing 60 % by mol or more of vinylpyridine are preferable in order to give a high microbial adsorbability.

A vinylpyridine polymer useful in the present invention preferably has a molecular weight of approximately 1000 to 1,000,000.

These vinylpyridine polymers may be prepared by, for example, known solution or suspension polymerization techniques.

The quaternization of the vinylpyridine polymer with the molded halomethyl polymer may be carried out by contacting a solution of the vinylpyridine polymer in a solvent such as an alcohol (e.g., methanol, ethanol or propanol), chloroform, dichloromethane, N,N-dimethylformamide, N,N-dimethylacetamide or dimethylsulfoxide with the molded halomethyl polymer. These materials can be contacted each other by suspending the molded halomethyl polymer in the solution of the vinylpyridine polymer or passing the vinylpyridine polymer solution through a container packed with the molded halomethyl polymer.

The abovementioned reaction between the molded halomethyl polymer and the vinylpyridine polymer may be conducted at a temperature commonly employed in the quaternization of pyridine, for example, 40 to 100 °C.

The unreacted nitrogen atom of pyridine ring of the vinylpyridine polymer thus carried on the surface of the molded polymer is then quaternized through a reaction with a known quaternizing agent, for example, a benzyl halide such as benzyl chloride, benzyl bromide or benzyl iodide, or the substituted aromatic nucleus derivatives thereof; a phenethylethyl halide such as phenethyl chloride, phenethylbromide or phenethyl iodide or the substituted aromatic nucleus derivative thereof; pentafluorophenylmethyl halide such as pentafluorophenylmethyl chloride, pentafluorophenylmethyl bromide or pentafluorophenylmethyl iodide; an alkyl halide having 1 to 16 carbon atoms; an alcohol disulfate having 1 to 16 carbon atoms; a p-toluenesulfonate; or a trifluoromethanesulfonate under conditions allowing the quaternization of pyridines.

Different from the quaternization of a crosslinking vinylpyridine polymer, the quaternization of the vinylpyridine polymer carried on the surface of the molded polymer would easily proceed since it is not affected by the diffusion of the quaternizing agent into the inside of the polymer.

Some portion of the vinylpyridine polymer may be preliminarily quaternized with the abovementioned alkylating agent prior to the reaction of the vinylpyridine polymer with the molded polymer having a halomethyl group. However this quaternization should be limited to such a degree that the subsequent reaction with the molded halomethyl polymer is not inhibited thereby.

Among the microbial adsorbents thus obtained, those which have benzyl, phenylethyl, ethyl and/or methyl groups as substituents on the nitrogen atom of the pyridinium group are particularly preferable because of their high microbial adsorbabilities.

The microbial adsorbent of the present invention may by in various forms such as granules, a powder, a mass, fibers, a fabric, a film or a sponge depending on the form of the molded polymer employed as the carrier. Furthermore, the properties of the microbial adsorbent may be widely varied, for example, either flexible or rigid or either hydrophilic or hydrophobic, depending on the purpose desired by selecting an appropriate carrier.

The microbial adsorbent of the present invention thus obtained has a high absorption effect on various microorganisms such as bacteria, fungi, algae, viruses and protozoa. Therefore it is useful, for example, in removing microorganisms present in the atmosphere or water. The practical application thereof may be carried out by, for example, contacting the abovementioned microbial adsorbent with water, various aqueous solutions or dispersions, the atmosphere, or various gases or the surface of various organic or inorganic solids containing microorganisms so as to remove of said microorganisms. In this case, either one or two or more the microbial adsorbents may be used. Furthermore, known bactericide(s) and/or antibacterial agent(s) may be used together with the microbial adsorbent of the present invention.

Accordingly, the microbial adsorbent of the present invention may be effectively applied to the removal of microorganisms from, for example, drinking water, treating waters for the production of, for example, foods, drugs or cosmetics, swimming pool, chemical industrial waters for pulp or fermentation plants, various cooling waters, various waste waters and waters treating the same. Further, microbial contamination of air in, for example, sickrooms, operation rooms, culture plants for microorganisms and precision machinery plants may be prevented by, for example, passing the air through a container packed with the abovementioned microbial adsorbent. Furthermore, the microbial adsorbent is available in the prevention of the growth of microorganisms on the surface of solids such as sanitary goods, buildings and public transportation facilities.

Any weakly basic anion exchange resin may be employed as the microbial adsorbent (b) of the microbial sensor of the present invention, so long as it has primary to tertiary amino group(s) as ion exchange group(s). It may have either a single amino group or two or more ones as the ion exchange groups.

Commonly marketed weakly basic anion exchange resins, such as IRA-94 (trade name, manufactured by Organo Co., Ltd.), WA-30E (trade name, manufactured by Mitsubishi Kasei Corporation) or Dow X-66 (trade name, Dow Chemicals Corp.), may be useful as the microbial adsorbent of the present invention.

Although anion exchange resins involve weakly basic ones and strongly basic ones, the latter are not useful in the microbial sensor of the present invention. Thus a weakly basic anion exchange resin is to be exclusively employed as the microbial adsorbent in the present invention.

When the microbial adsorbent of the present invention is to be used as a carrier for the preparation of immobilized cells for a microbial sensor, it may be in the form of spheres, an amorphous mass, fibers or a film, without restriction. However, spheres of a particle size of 0.01 to 5 mm, in particular, 0.1 to 1 mm are preferable. Furthermore porous spheres having a number of voids in the interior or on the surface thereof may be employed.

The electrochemical device which can be used in the present invention is a device which can determine a material formed or consumed under the effects of the microorganisms. Examples and details of the electrochemical device which can be used in the present invention are disclosed, for example, in Kagaku Sensor, Sono Kiso to Ouyou, published by Kodansha Scientific, page 187 (March 1982).

Now, the measurement of BOD with the use of the microbial sensor of the present invention of the abovementioned type will be illustrated by reference to Fig. 2 which by way of example is where an oxygen electrode is used as an electrode.

In Fig. 2, 6 is a resin container packed with a microbial adsorbent 16, and 7 is an electrode and the result of the measurement with it is recorded in a recorder 12. The BOD of a sample 13 may be measured by using this microbial sensor in the following manner. First, changing valves 10 and 11 are adjusted in such a manner as to allow a microbial suspension 14 to circulate in the resin container 6 packed with the microbial adsorbent. The microbial suspension is fed with a pump 9 and microorganisms contained therein are adsorbed and immobilized on the microbial adsorbent 16. When this microbial sensor is to be used as a BOD sensor, any microbial suspension may be employed so long as it contains aerobic microorganism(s).

Examples thereof include a suspension in an activated sludge tank or river water. The adsorption of a microorganism may be conducted by feeding a microbial suspension into a container packed with a resin, as described above. Alternately, a microbial adsorbent may be added to a microbial suspension and shaken or stirred therein for a definite period of time to thereby allow it to adsorb a microorganism. Then the microbial adsorbent is taken out and packed in a resin container.

Next, the changing valves 10 and 11 are adjusted so as to feed a buffer solution until the dissolved oxygen concentration of the solution passing through the resin container 6 becomes stable and a certain base line is achieved. The buffer solution may be replaced with deionized water so long as the replacement exerts no undesirable effect on the microorganism.

It is preferable to provide to the resin container 6, the measuring flow cell 8, the sample 13 and the buffer solution or deionized water 15 in a thermostat, and to preliminarily aerate the sample 13 and the buffer solution 15 by an air pump 23.

Then the changing valve 11 is adjusted so as to feed a definite amount of the sample. The dissolved oxygen concentration loss and the decreasing rate thereof are measured and the BOD value is determined from the calibration curve which has been preliminarily formed.

The time required for a single measurement varies depending on the microorganism to be adsorbed, the amount of the adsorbed microorganism, and the operation conditions such as the amount of the packed microbial adsorbent and the flow rate. When microorganisms in an activated sludge tank are to be adsorbed by using a resin container of 2.5 ml in volume, for example, a single measurement may be completed within 5 to 10 minutes by using 0.5 ml of a sample at a flow rate of 5 ml/min. In the case of a sample of a high concentration, i.e., showing a BOD higher than 500, the dissolved oxygen concentration returns to the base line within 10 minutes, and the measuring can be completed within 10 minutes.

A microbial sensor showing in Fig. 6 is another example of an embodiment of the present invention, whereby a sample injected in a definite amount can be analyzed. First, a definite amount of the sample is packed in a sample loop 17 with a pump 22. At this stage, a buffer solution is passed and thus a base line achieved. Next, changing valves 18 to 21 are adjusted so as to extrude the sample 13 with the buffer solution. Then the change in the dissolved oxygen concentration of the sample thus injected into a resin container 6 is monitored.

The microbial sensor of the present invention is available not only in the measurement of BOD but also in the determination of various substances consumed or produced by microorganisms, by appropriately selecting the microorganism to be adsorbed by the microbial adsorbent and selecting an appropriate electrochemical device.

For example, a glucose sensor can be prepared by combining an adsorbed and immobilized microorganism capable of metabolizing glucose such as Pseudomonas fluoresens with an oxygen electrode. Further an ammonia sensor can be obtained by combining an adsorbed and immobilized microorganism capable of oxidizing ammoniacal nitrogen into nitrate nitrogen such as Nitrosomonas or Nitrobacter with an oxygen electrode. Furthermore, an amino acid sensor can be obtained by combining Escherichia coli having a glutamate decarboxylase activity with a carbon dioxide electrode. These are specific examples of microbial sensor of the present invention, but other various microbial sensors satisfying the requirements of the present invention can also be prepared, and are considered encompassed herein.

It is a characteristic of the present invention that the concentration of the target substance can be rapidly measured at a high reproducibility, since the container packed with the microbial adsorbent, on which a microorganism is immobilized, is separately located from the electrochemical device and connected to it via a pipe and solutions are forced to pass through the container with, for example, a pump. It is another characteristic of the present invention that a microorganism suitable for the purpose and an appropriate electrochemical device can be selected without requiring any troublesome immobilization procedure, since the microbial adsorbent employed as a carrier in the immobilization of the microorganism is capable of intensely adsorbing a large amount of a living microorganism. Thus the microbial sensor of the present invention can be readily applied to various purposes.

According to the microbial sensor of the present invention, therefore, it is possible to rapidly and readily determine not only the BOD of an aqueous solution but also various substances such as sugars, alcohols and organic acids.

Now the present invention will be described in detail in the following Examples and Synthesis Examples. However it is to be understood that the present invention is not intended to be limited thereto.

## Synthesis Example 1

Synthesis of crosslinking poly(1-methyl-4-vinylpyridinium) chloride:

(i) 500 g of water was introduced into a 1 ℓ separable flask provided with a stirrer, a condenser, a thermometer and a nitrogen inlet. 10 g of a calcium carbonate powder was added thereto and homogeneously dispersed by stirring at 150 rpm. Further a solution comprising 105 g of 4-vinylpyridine, 13 g of divinylbenzene, 1 g of azoisobutylonitrile and 100 g of isoamyl alcohol was added thereto and the obtained mixture was heated to 80 °C for 3 hours while stirring at 150 rpm. The obtained polymer particles were filtered and washed with 1 % acetic acid to thereby dissolve and remove the calcium carbonate. Then these particles were repeatedly washed with water and 1 % sodium bicarbonate and finally with ethanol. After drying in vacuum, a 4-vinylpyridine/divinylbenzene copolymer was obtained in the form of spherical particles. Yield: 105 g; Average particle size of dry copolymer: 200μm. Nitrogen content: 11 %.

(ii) 21 g of the copolymer synthesized in the above (i) was heated to 50 °C in a container, capable of withstanding heating under pressure or reduced pressure, under a methyl chloride atmosphere (2 atom) for 3 hours. Next, the methyl chloride gas was removed and the polymer was dried in vacuum at room temperature. Thus 25 g of crosslinking poly(1-methyl-4-vinylpyridinium) chloride, wherein the pyridine group in the product of (i) was converted into a N-methylpyridinium group (counter ion: chloride ion), was obtained in the form of particles. Chlorine content: 18 %.

## Synthesis Example 2

Synthesis of crosslinking poly(1-ethyl-4-vinylpyridinium) chloride:

21 g of the copolymer particles prepared in the above Synthesis Example 1-(i) were treated in the same manner as one described in Synthesis Example 1-(ii) except that the methyl chloride gas was substituted with ethyl chloride gas. Thus 27 g of crosslinking poly(1-ethyl-4-vinylpyridinium) chloride, wherein the pyridine group of the starting copolymer is converted into a N-ethylpyridinium group (counter ion: chloride ion, was obtained in the form of particles. Chlorine content: 16 %.

## Synthesis Example 3

Synthesis of crosslinking poly(1-methyl-4-vinylpyridinium) bromide:

21 g of the copolymer particles prepared in the above Synthesis Example 1-(i) were treated in the same manner as described in Synthesis Example 1-(ii) except that the methyl chloride gas was substituted with methyl bromide gas. Thus 31 g of crosslinking poly(1-methyl-4-vinylpyridinium) bromide, wherein the pyridine group of the starting copolymer is converted into a N-methylpyridinium group (counter ion: bromide ion), was obtained in the form of particles. Bromine content: 33 %.

## Synthesis Example 4

Synthesis of crosslinking poly(1-ethyl-4-vinylpyridinium) bromide:

21 g of the copolymer particles prepared in the above Synthesis Example 1-(i) were treated in the same manner as described in Synthesis Example 1-(ii) except that methyl chloride gas was substituted with an ethyl bromide gas. Thus 33 g of crosslinking poly(1-ethyl-4-vinylpyridinium) bromide, wherein the pyridine group of the starting copolymer is converted into a N-ethylpyridinium group (counter ion: bromide ion), was obtained in the form of particles. Bromine content: 31 %.

Synthesis Example 5

Synthesis of crosslinking poly(1-ethyl-4-vinylpyridinium) bromide:

10 g of the copolymer particles prepared in the above Synthesis Example 1-(i) were dispersed in 100 g of methanol and 33 g of ethyl bromide was added thereto at room temperature under atmospheric pressure. Then the reaction mixture was allowed to react at 50 °C under atmospheric pressure for 5 hours. The obtained polymer beads were filtered, isolated, washed with acetone and dried in vacuum. Thus 15 g of crosslinking poly(1-ethyl-4-vinylpyridinium) bromide, wherein the pyridine group of the starting copolymer is converted into a N-ethylpyridinium group (counter ion: bromide ion), was obtained in the form of particles. Bromine content: 30 %.

Synthesis Example 6

Synthesis of crosslinking poly(1-benzyl-4-vinylpyridinium) bromide:

10 g of the copolymer particles prepared in the above Synthesis Example 1-(i) were treated in the same manner as described in Synthesis Example 5 except that the ethyl bromide was substituted with 51 g of benzyl bromide. Thus 20 g of crosslinking poly(1-benzyl-4-vinylpyridinium) bromide, wherein the pyridine group of the starting copolymer is converted into a N-benzylpyridinium group (counter ion: bromide ion), was obtained in the form of particles. Bromine content: 25 %.

Synthesis Example 7

Synthesis of crosslinking poly(1-phenethyl-4-vinylpyridinium) bromide:

(i) 500 g of water was introduced into a 1 ℓ separable flask provided with a stirrer, a condenser, a thermometer and a nitrogen inlet. 10 g of a calcium carbonate powder was added thereto and homogeneously dispersed by stirring at 150 rpm. Further a solution comprising 105 g of 4-vinylpyridine, 10 g of styrene, 13 g of divinylbenzene, 1 g of azoisobutylonitrile and 100 g of isoamyl alcohol was added thereto and the obtained mixture was heated to 80 °C for 3 hours while stirring at 150 rpm. The obtained polymer beads were filtered and washed with 1 % acetic acid to thereby dissolve and remove the calcium carbonate. Then these particles were repeatedly washed with water and 1 % sodium bicarbonate and finally with ethanol. After drying in vacuum, a 4-vinylpyridine/styrene/divinylbenzene copolymer was obtained in the form of spherical particles. Yield: 110 g. Average particle size of dry copolymer: 200 µm. Nitrogen content: 10 %. This copolymer is referred to as "vinylpyridine polymer (1)."

(ii) 21 g of the vinylpyridine polymer (1) was suspended in 100 g of methanol and 120 g of phenethyl bromide was added thereto. The obtained mixture was heated to 60 °C for 5 hours. The polymer beads were filtered, isolated, washed with ethanol and dried in vacuum. Thus 40 g of the title polymer, wherein the pyridine group in the polymer (1) was converted into a N-phenethylpyridinium group (counter ion: bromide ion), was obtained in the form of particles. Yield: 40 g. Bromine content: 23 %.

Synthesis Example 8

Synthesis of crosslinking poly(1-phenethyl-4-vinylpyridium) bromide:

(i) The procedure of Synthesis Example 7-(i) was repeated except that no styrene was employed to

thereby give a 4-vinylpyridine/divinylbenzene copolymer in the form of particles. Yield: 105 g. Average particle size of dry copolymer: 200 µm. Nitrogen content: 11 %. This copolymer was referred to as vinylpyridine polymer (2).

(ii) The pyridine groups of 21 g of the vinylpyridine polymer (2) were converted into N-phenethylpyridinium groups (counter ion: bromide ion) in the same manner as described in Synthesis Example 7-(ii). Thus the title polymer was obtained. Yield: 38 g. Nitrogen content: 25 %.

## Synthesis Example 9

Synthesis of crosslinking poly(1-benzyl-4-vinylpyridium) bromide:

21 g of the vinylpyridine polymer (2) prepared in Synthesis Example 8-(i) was treated in the same manner as described in Synthesis Example 7-(ii) except that phenethyl bromide was replaced with benzyl bromide. Thus the title polymer, wherein the pyridine group of the vinylpyridine polymer (2) was converted into a N-benzylpyridinium group (counter ion: bromide ion), was obtained. Yield: 39 g. Nitrogen content: 24 %.

## Synthesis Example 10

Synthesis of polymer beads carrying poly(1-phenethyl-4-vinylpyridinium) bromide:

(i) A solution comprising 20 g of p-chloromethylstyrene, 70 g of styrene, 10 g of divinylbenzene and 1 g of lauroyl peroxide, 380 g of water and 23 g of polyvinyl alcohol (Gosenol GH-17, trade name, manufactured by Nippon Gosei Kagaku Kogyo K.K.) were introduced into a 1 ℓ separable flask provided with a stirrer, a condenser, a thermometer and a nitrogen inlet. The mixture was heated to 80 °C under stirring at 200 rpm for 8 hours. The polymer beads thus obtained were filtered, successively washed with water and acetone and dried in vacuum. Thus crosslinking chloromethylstyrene/styrene copolymer beads (hereinafter referred to as a "haloalkylated polymer (1)") were obtained. Yield: 95 g. Average particle size of polymer beads: 300µm. Chlorine content: 4.6 %.

(ii) 10 g of the haloalkylated polymer obtained in the above (i) were suspended in a solution comprising 10 g of poly(4-vinylpyridinium) (number-average molecular weight: 50,000) and 100 g of methyl alcohol and heated under reflux for 5 hours. The reaction mixture was filtered and the obtained polymer beads were washed by Soxhlet extraction by using methanol as a solvent. The washed polymer beads were then dried in vacuum to thereby give polymer beads carrying polyvinylpyridine. Yield: 9.8 g. Nitrogen content: 1.2 %. 8.0 g of the polyvinylpyridine-carrying polymer beads thus obtained were suspended in a solution comprising 8.0 g of benzyl bromide and 50 g of methyl alcohol and heated under reflux for 5 hours. The reaction mixture was filtered and the obtained polymer beads were washed with acetone and dried in vacuum . Thus polymer beads carrying poly(N-benzylvinylpyridinium) bromide were obtained. Yield: 9.25 g. Bromine ion content: 6.5 %. It was also found that the polymer beads obtained carried 290 mg of poly(N-benzylvinylpyridinium) bromide per gram of the haloalkylated polymer.

## Synthesis Example 11

The procedure of Synthesis Example 10-(ii) was repeated except that the poly(4-vinylpyridinium) (number-average molecular weight: 50,000) was replaced with poly(4-vinylpyridinium) (number average molecular weight: 1,000), and polymer beads of a nitrogen content of 0.40 % were obtained. 8.0 g of the polymer beads thus obtained were suspended in a solution comprising 8.0 g of ethyl bromide and 50 g of methyl alcohol and heated under reflux for 5 hours. The reaction mixture was filtered and the obtained polymer beads were washed with acetone and dried in vacuum. Thus polymer beads carrying poly(N-ethylvinylpyridinium) bromide were obtained. Yield: 8.2 g. Bromine ion content: 2.2 %. It was found that the

polymer beads prepared carried 63 mg of poly(N-ethylvinylpyridinium) bromide per gram of the haloalkylated polymer (1).

Synthesis Example 12

The procedure of Synthesis Example 10-(ii) was repeated except that the poly(4-vinylpyridinium) (number-average molecular weight: 50,000) was replaced with poly(4-vinylpyridinium) (number average molecular weight: 200,000), and polymer beads of a nitrogen content of 0.80 % were obtained. 8.0 g of the polymer beads thus obtained were suspended in a solution comprising 10.0 g of phenethyl bromide and 50 g of methyl alcohol and heated under reflux for 5 hours. The reaction mixture was filtered and the obtained polymer beads were washed with acetone and dried in vacuum. Thus polymer beads carrying poly(N-phenethylvinylpyridinium) bromide were obtained. Yield: 8.7 g. Bromine ion content: 4.1 %. It was found that of the polymer beads prepared carried 177 mg of poly(N-phenethylvinylpyridinium) bromide per gram of the haloalkylated polymer (1).

Synthesis Example 13

Synthesis of polymer beads carrying poly(N-benzylvinylpyridinium)chloride:

(i) A solution comprising 20 g of dimethylaminoethyl methacrylate, 70 g of styrene, 10 g of divinylbenzene and 1 g of azobisisobutylonitrile was mixed with 380 g of water and 2.3 g of polyvinyl alcohol (Gosenol GH-17) and the obtained mixture was stirred and heated to 65 °C for 8 hours in the same manner as described in Synthesis Example 10-(i). The polymer beads thus obtained were subjected to the same post-treatment as described in Synthesis Example 1, and then the beads isolated. Yield: 90 g. Average particle size of polymer beads: 300µm. Nitrogen content: 1.6 %.

Next, 50 g of these polymer beads were suspended in 200 g of isopropyl alcohol and 30 g of 1,3-dibromopropane was added thereto. The mixture was heated to 80 °C for 8 hours. Then the reaction mixture was filtered and the polymer beads were washed with acetone and dried in vacuum. Thus polymer beads having a 3-bromopropyl-dimethylamonio group (hereinafter referred to as a "haloalkylated polymer (2)") were obtained. Yield: 51 g. Bromine ion content: 5.2 %. Covalent bound nitrogen content: 5.2 %.

(ii) 10 g of the haloalkylated polymer (2) obtained in the above (i) was treated in the same manner as the one described in Synthesis Example 10-(ii) to thereby give polymer beads carrying polyvinylpyridine. Yield: 9.5 g. Nitrogen content: 1.8 %.

Next, 8.0 g of the polyvinylpyridine-carrying polymer beads were suspended in a solution comprising 8.0 g of benzyl chloride and 50 g of methyl alcohol and heated under reflux for 5 hours. The reaction mixture was filtered and the obtained polymer beads were washed with acetone and dried in vacuum. Thus polymer beads carrying poly(N-benzylvinylpyridinium) chloride were obtained. Yield: 8.3 g. Chlorine ion content: 0.49 %. Thus it was found that the polymer beads carrying 41 mg of poly(N-benzylvinylpyridinium) chloride per gram of the haloalkylated polymer (2) were obtained.

Synthesis Example 14

Synthesis of polymer carrying poly(N-decylvinylpyridinium) bromide:

(i) 50 g of a polyvinyl alcohol fiber was added to 200 mℓ of a mixture comprising 200 g/ℓ of sodium sulfate, 200 g/ℓ of sulfuric acid and 40 g/ℓ of chloroacetaldehyde and heated to 50 °C for 30 minutes. After the completion of the reaction, the fiber was taken out, washed with water and dried in vacuum. Thus a fiber acetalated with chloroacetaldehyde (hereinafter referred to as a "haloalkylated polymer (3)") was obtained. Yield: 53 g. Chlorine content: 5.0 %.

(ii) 10 g of the haloalkylated polymer (3) obtained in the above (i) was treated in the same manner as

described in Synthesis Example 10-(ii) to thereby give polymer beads carrying polyvinylpyridine. Yield: 10.5 g. Nitrogen content: 0.62 %.

Next, 8.0 g of the polyvinylpyridine-carrying polymer was suspended in a solution comprising 8.0 g of decyl bromide and 50 g of methyl alcohol and heated under reflux for 5 hours. The reaction mixture was filtered and the obtained polymer was washed with acetone and dried in vacuum. Thus a polymer carrying poly(N-decylvinylpyridinium) bromide was obtained. Yield: 8.5 g. Bromine ion content: 3.2 %. Thus it was found that the polymer obtained carried 116 mg of poly(N-decylvinylpyridinium) bromide per gram of the haloalkylated polymer (3).

## Synthesis Example 15

Synthesis of fabric carrying poly(N-benzylvinylpyridinium) bromide:

(i) 50 g of a cotton fabric was suspended in a solution comprising 350 g of acetic anhydride, 100 g of bromoacetic acid and a catalytic amount of sodium acetate and heated to 35 °C for 5 hours. After the completion of the reaction, the fabric was taken out, washed with acetone and dried in vacuum. Thus a bromoacetylated cotton fabric (hereinafter referred to as "haloalkylated polymer (4)") was obtained. Yield: 50.5 g. Covalent bromine content: 3.3 %.

(ii) 30 g of the haloalkylated polymer (4) (fabric) obtained in the above (i) was added to a solution comprising 10 g of poly(4-vinylpyridine) (number-average molecular weight: 50,000) and 200 g of methyl alcohol and heated under reflux for 5 hours. Next, the fabric was taken out from the reaction mixture and washed by Soxhlet extraction by using methanol as a solvent. After washing, the fabric was dried in vacuum to thereby give a fabric carrying polyvinylpyridine. Yield: 31.1 g. Nitrogen content: 0.32 %.

Next, 10 g of the polyvinylpyridine-carrying fabric was suspended in a solution comprising 8.0 g of benzyl bromide and 50 g of methyl alcohol and heated under reflux for 5 hours. The fabric was taken out from the reaction mixture and washed with acetone and dried in vacuum. Thus a fabric carrying poly(N-benzylvinylpyridinium) bromide was obtained. Yield: 10 g. Bromide ion content: 1.7 %. It was found that the fabric obtained carried 80 mg of poly(N-benzylvinylpyridinium) bromide per gram of the haloalkylated polymer (4).

## Synthesis Example 16

Synthesis of fabric carrying poly(N-methylvinylpyridinium) chloride:

(i) 10 g of the fabric carrying polyvinylpyridine prepared in Synthesis Example 15-(ii) and 50 g of methyl chloride were introduced into an autoclave and heated to 60 °C for 8 hours. After the completion of the reaction, the reaction mixture was dried under reduced pressure as such to thereby give a fabric carrying poly(N-methylvinylpyridium) chloride. Yield: 10 g. Chlorine ion content: 0.80 %. It was found that the fabric obtained carried 50 mg of poly(N-methylvinylpyridium) chloride per gram of the haloalkylated polymer (4).

## Synthesis Example 17

A slurry comprising 25 g of calcium phosphate and 200 g of deionized water and a solution comprising 42 g of 4-vinylpyridine, 10.5 g of divinylbenzene, 30 g of chlorobenzene, 20 g of 1-butanol and 0.3 g of azobisisobutylonitrile were introduced into a 1 ℓ separable flask provided with a stirrer, a condenser, a thermometer and a nitrogen inlet. The mixture was polymerized by heating to 75 °C for 5 hours under stirring at 300 rpm. After cooling, the obtained polymer beads were filtered and then washed with ethanol. Next, the polymer beads were added to a solution comprising 150 g of ethanol and 60 g of benzyl bromide and heated to 70 °C for 5 hours. The reaction product was filtered, washed with ethanol and dried under

reduced pressure. Then the product was washed with a 0.1 N aqueous solution of sodium hydrogencarbonate and then with deionized water to thereby give crosslinking polymer beads (1). Yield: 45 g. The product was in the form of spheres having an average particle size of 400 µm.

## Synthesis Example 18

A slurry comprising 25 g of calcium carbonate and 200 g of deionized water and a solution comprising 38.8 g of 4-vinylpyridine, 10.5 g of divinylbenzene, 5.2 g of styrene, 30 g of chlorobenzene, 20 g of 1-butanol and 0.3 g of azobisisobutylonitrile were introduced to the same apparatus as used in Synthesis Example 17. The mixture was polymerized in the same manner as the one described in Synthesis Example 17. Next, the obtained polymer was reacted with benzyl bromide in the same manner as described in Synthesis Example 17 to thereby give crosslinking polymer beads (2). Yield: 43 g. The product was in the form of spheres having an average particle size of 350 µm.

## Synthesis Example 19

40 g of 4-vinylpyridine/divinylbenzene copolymer beads synthesized in the same manner as described in Synthesis Example 17 were added to a solution comprising 150 g of ethanol and 70 g of 3-phenyl-1-bromopropane and heated to 70 °C for 5 hours. The product was filtered, washed with ethanol and dried under reduced pressure. Next, the product was successively washed with a 0.1 N aqueous solution of sodium hydrogencarbonate and deionized water to thereby give crosslinking polymer beads (3). Yield: 50 g. The product was in the form of spheres having an average particle size of 400 µm.

## Synthesis Example 20

40 g of 4-vinylpyridine/styrene/divinylbenzene copolymer beads synthesized in the same manner as described in Synthesis Example 18 were added to a solution comprising 150 g of ethanol and 30 g of methyl bromide and reacted at 10 °C for 24 hours. Volatile material was removed from the reaction mixture under reduced pressure and the residual product was successively washed with a 0.1 N aqueous solution of sodium hydrogencarbonate and deionized water to thereby give crosslinking polymer beads (4). Yield: 43 g. The product was in the form of spheres having an average particle size of 400 µm.

## Synthesis Example 21

The procedure of Synthesis Example 17 was repeated except that the calcium phosphate slurry was replaced with 200 g of a 0.3 % aqueous solution of polyvinyl alcohol (saponification degree: 86.5 %) to thereby give crosslinking polymer beads (5).

## EXAMPLE 1

A BOD sensor was formed by using the system shown in Fig. 2. An oxygen electrode (GU-BP, trade name, manufactured by Iijima Denki Kogyo Co., Ltd.) was employed as an electrode. A resin container of 2.5 mℓ in volume was packed with crosslinking poly-(1-benzyl-4-vinylpyridinium) bromide beads obtained in Synthesis Example 6. The changing valves 10 and 11 were adjusted in such a manner that a microbial suspension 14 would circulate in the system in the order of 14 ⇒ 11 ⇒ 9 ⇒ 6 ⇒ 10 ⇒ 14. Next, the microbial suspension was fed by using a pump 9 at a flow rate of 2.5 mℓ/min for 30 minutes to thereby adsorb and immobilize microorganisms. In this Example, a suspension in an activated sludge tank was employed as the microbial suspension.

Then the changing valves were adjusted in such a manner that a buffer solution 15 would flow toward a measuring flow cell 8 via the resin container 6. The buffer solution was passed until the dissolved oxygen concentration became stable and a certain base line was obtained.

A sample solution of a known BOD was prepared and a calibration curve showing the relationship between BOD and dissolved oxygen concentration was formed, by using the same sample. At the start of the operation, the buffer solution was fed at a flow rate of 2.5 mℓ/min. Then the valve 11 was adjusted so as to feed 0.2 mℓ of the sample, and then the valve 11 was adjusted again and the buffer solution was again fed. In this Example, the measuring flow cell 8, the resin container 6, the sample 13 and the buffer solution 15 were each kept at 20 °C.

Fig. 3 shows the relationships between time and dissolved oxygen concentration in the analysis of samples having BODs of 120, 240 and 560 ppm. Fig. 3 indicates that the dissolved oxygen concentration would return to the base line within 10 minutes, which enables the subsequent analysis, in each case. Fig. 4 show the relationship between the reciprocal of dissolved oxygen concentration loss (ΔDO) and the reciprocal of BOD. The graph shows an excellent linear relationship. Namely, the BOD of a sample to be analyzed can be determined by feeding the sample, measuring the dissolved oxygen concentration and reading the aimed at value by using a calibration curve which has been preliminarily formed with the aid of a solution having a known BOD.

Table 1 shows BOD values determined by the BOD sensor employed in this Example and those determined according to a method specified in JIS K0102.

Table 1

| Sample No. | BOD determined by microbial sensor of invention | BOD determined by JIS method |
|---|---|---|
| | (mg/ℓ) | (mg/ℓ) |
| 1 | 371 | 360 |
| 2 | 182 | 180 |
| 3 | 147 | 150 |
| 4 | 72 | 75 |
| 5 | 548 | 560 |
| 6 | 187 | 195 |
| 7 | 97 | 97 |
| 8 | 86 | 80 |

These results indicate that the data obtained using the BOD sensor of the present invention agreed well with those determined by the JIS method. The determination of BOD by the JIS method requires a long period, i.e., 5 days and a complicated procedure which should be conducted by a skilled person. Whereas, the application of the microbial sensor of the present invention makes it possible to readily and rapidly determine BOD at a high accuracy.

## EXAMPLE 2

A change in dissolved oxygen concentration was monitored in the same manner as the one described in Example 1 and the relationship between BOD and the initial decreasing rate of dissolved oxygen concentration was examined. The flow rate was 4 mℓ/min and 1 mℓ of a sample was fed. Fig. 5 shows the result. This result indicates that an excellent linear relationship is observed between the reciprocal of BOD and the initial decreasing rate of dissolved oxygen concentration. Thus it is also possible to determine the aimed at BOD from the initial decreasing rate of dissolved oxygen concentration.

## EXAMPLE 3

A system shown in Fig. 6 was employed in order to feed a definite amount of a sample. In this system, a definite amount of the sample was packed in a sample loop 17. Then the changing valves 18 to 21 were adjusted so as to extrude the sample with a buffer solution. After adsorbing microorganisms in the same manner as described in Example 1, a sample loop of 0.2 mℓ in volume was provided and a buffer solution was fed at a flow rate of 2.5 mℓ/min. Thus the relationship between BOD and ΔDO was examined. Fig. 7 shows the results obtained, namely, an excellent linear relationship was observed between 1/BOD and 1/ΔDO.

## Example 4

A glucose sensor was formed by using the system shown in Fig. 2. The same operation procedure as described in Example 1 was employed, except that a microbial adsorbent on which Pseudomonas fluorescens was immobilized was employed. Fig. 8 shows the relationship between the reciprocal of glucose concentration and the reciprocal of dissolved oxygen concentration loss (ΔDO). Fig. 8 obviously indicates that an excellent linear relationship is observed between these factors. Thus the microbial sensor of the present invention is applicable as a glucose sensor.

Table 2 shows the comparison of the actual glucose concentrations and those determined by the glucose sensor described above. As can be seen, the two data columns well agree very well with each other.

Table 2

| Glucose concentration determined by Microbial sensor of the Invention | Actual glucose concentration |
|---|---|
| (mg/ℓ) | (mg/ℓ) |
| 500 | 500 |
| 1002 | 1000 |
| 2008 | 2000 |
| 4995 | 5000 |
| 9967 | 10000 |
| 20082 | 20000 |

## EXAMPLE 5

A microbial sensor as shown in Fig. 9 was formed in order to determine the BOD of a sample prior to a microbial reaction based on the dissolved oxygen concentration In the case of this microbial sensor, the adsorption and immobilization of a microorganism is first conducted by using a pump 9 in a method similar to that employed Example 1. Then the sensor was to operated in the following manner. The flow rate of each solution was 5 mℓ/min.

Procedure 1:

Two lines, namely, 13 ⇒ 26 ⇒ 25 ⇒ 8 ⇒ drain and 15 ⇒ 11 ⇒ 9 ⇒ 6 ⇒ 24 ⇒ drain were formed by adjusting the changing valves 11, 24 and 25. A sample and a buffer solution were fed respectively from pumps 26 and 9. The dissolved oxygen concentration at this stage was the dissolved oxygen concentration of the sample ($DO_1$).

Procedure 2:

The operation of the pump 26 was stopped and the changing valves were adjusted to thereby form a line 15 ⇒ 11 ⇒ 9 ⇒ 6 ⇒ 24 ⇒ 25 ⇒ 8 ⇒ drain. The buffer solution was fed and the dissolved oxygen concentration of the same ($DO_0$) was measured.

Procedure 3:

The changing valves were adjusted to thereby form a line 13 ⇒ 11 ⇒ 9 ⇒ 6 ⇒ 24 ⇒ 25 ⇒ 8 ⇒ drain. 1 mℓ of the sample was fed and then the system was returned to the conditions of the above Procedure 2. The minimum dissolved oxygen concentration ($DO_3$) was then measured.

Various samples were analyzed by repeating these procedures and the relationship between dissolved oxygen concentrtion los ($DO_1$ - $DO_3$) and BOD was examined. Fig. 10 shows the results. Fig. 10 shows that an excellent linear relationship is observed between 1/($DO_1$ - $DO_3$) and 1/BOD.

## EXAMPLE 6

A resin container was packed with the crosslinking poly(1-phenethyl-4-vinylpyridinium) bromide beads (average particle size: 0.3 mm) obtained in Synthesis Example 7 and the relationship between BOD and ΔDO was examined under the same conditions as those employed in Example 1. As shown in Fig. 11, an excellent linear relationship was observed between 1/BOD and 1/ΔDO. Accordingly, the BOD of an unknown sample can be determined by measuring the ΔDO thereof.

## EXAMPLE 7

A resin container was packed with the crosslinking poly(1-methyl-4-vinylpyridinium) bromide beads (average particle size: 0.3 mm) obtained in Synthesis Example 3 and the relationship between BOD and ΔDO was examined under the same conditions as those employed in Example 1. As shown in Fig. 12, an excellent linear relationship was observed between 1/BOD and 1/ ΔDO. Accordingly, the BOD of an unknown sample can be determined by measuring the ΔDO thereof.

## EXAMPLE 8

A resin container was packed with the crosslinking poly(1-ethyl-4-vinylpyridinium) bromide beads (average particle size: 0.3 mm) obtained in Synthesis Example 4 and the relationship between BOD and ΔDO was examined under the same conditions as those employed in Example 1. As shown in Fig. 13, an excellent linear relationship was observed between 1/BOD and 1/ΔDO. Accordingly, the BOD of an unknown sample can be determined by measuring the DO thereof.

## EXAMPLE 9

A resin container was packed with the crosslinking poly(1-octyl-4-vinylpyridinium) bromide beads (average particle size: 0.3 mm) synthesized by following the manner disclosed in Synthesis Example 1 and the relationship between BOD and ΔDO examined under the same conditions as those employed in Example 1. As shown in Fig. 14, an excellent linear relationship was observed between 1/BOD and 1/ΔDO. Accordingly, the BOD of an unknown sample can be determined by measuring the ΔDO thereof.

## EXAMPLE 10

A resin container was packed with the crosslinking poly(1-benzyl-4-vinylpyridinium) chloride beads (average particle size: 0.3 mm) synthesized by following the manner disclosed as in Synthesis Example 6 and the relationship between BOD and ΔDO examined under the same conditions as those employed in Example 1. As shown in Fig. 15, an excellent linear relationship was observed between 1/BOD and 1/ΔDO. Accordingly, the BOD of an unknown sample can be determined by measuring the ΔDO thereof.

## EXAMPLE 11

A resin container was packed with the crosslinking poly(1-pentafluorophenylmethyl-4-vinylpyridinium) bromide beads (average particle size: 0.3 mm) synthesized by following the manner disclosed in Synthesis Example 1 and the relationship between BOD and ΔDO was examined under the same conditions as those employed in Example 1. As shown in Fig. 16, an excellent linear relationship was observed between 1/BOD and 1/ΔDO. Accordingly, the BOD of an unknown sample can be determined by measuring the ΔDO thereof.

## EXAMPLE 12

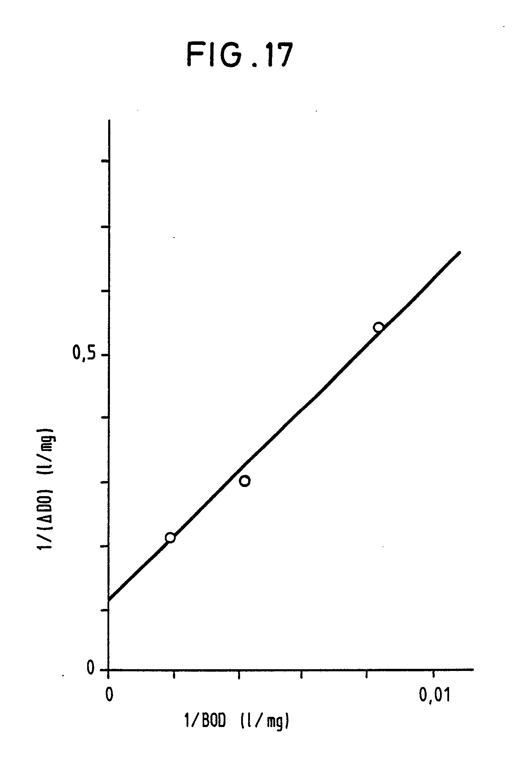

A resin container was packed with the polymer beads carrying crosslinking poly(1-benzyl-4-vinylpyridinium) bromide (average particle size: 0.3 mm), which had been prepared by quaternizing crosslinking chloromethylstyrene/styrene copolymer beads carrying poly(4-vinylpyridine) with benzyl bromide as shown in Synthesis Example 10, and the relationship between BOD and DO was examined under the same conditions as those employed in Example 1. As shown in Fig. 17, an excellent linear relationship was observed between 1/BOD and 1/ΔDO. Accordingly, the BOD of an unknown sample can be determined by measuring the ΔDO thereof.

## EXAMPLE 13

A BOD sensor was formed as in Example 1 except that the polymer beads obtained in Synthesis Example 6 were replaced with the tertiary amine type weakly basic anion exchange rein (IRA-94, trade name, manufactured by Organo Co., Ltd.), and BOD values of samples were measured.

Table 3 shows BOD values of several samples determined by the BOD sensor employed in this Example and those determined according to a method specified in JIS K0102.

Table 3

| Sample No. | BOD determined by microbial sensor of invention | BOD determined by JIS method |
|---|---|---|
| | (mg/ℓ) | (mg/ℓ) |
| 1 | 306 | 315 |
| 2 | 222 | 218 |
| 3 | 273 | 260 |
| 4 | 75 | 76 |
| 5 | 199 | 197 |
| 6 | 130 | 138 |
| 7 | 91 | 98 |
| 8 | 58 | 63 |

These results indicate that the data obtained by using the BOD sensor of the present invention agreed

well with those determined by the JIS method. The determination of BOD by the JIS method requires a long period, i.e., 5 days and a complicated procedure which should be conducted by a skilled person. On the other hand, the application of the microbial sensor of the present invention makes it possible to readily and rapidly determine BOD at a high accuracy.

EXAMPLE 14

Escherichia coli was suspended in a 0.85 % aqueous solution of common salt (NaCl) at a concentration of $5.0 \times 10^6$/mℓ. To 20 mℓ of the obtained suspension, was added 2.0 g of the microbial adsorption polymer obtained in Synthesis Example 10-(ii). The mixture was stirred at 150 rpm while being maintained at 37 °C. As a result, the bacterial count was lowered to $4.7 \times 10^5$/mℓ, $3.0 \times 10^4$/mℓ, $2.5 \times 10^3$/mℓ and $1.3 \times 10^2$/mℓ after 1, 2, 3 and 5 hours, respectively.

Example 15

Salmonella typhimurium was suspended in a 0.85 % aqueous solution of common salt (NaCl) at a concentration of $6.5 \times 10^6$/mℓ. To 20 mℓ of the obtained suspension, was added 2.0 g of the microbial adsorption polymer obtained in Synthesis Example 11. The mixture was stirred at 150 rpm while being maintained at 37 °C. As a result, the bacterial count was lowered to $5.8 \times 10^4$/mℓ, $4.2 \times 10^3$/mℓ and $1.2 \times 10^2$/mℓ after 1, 3 and 5 hours, respectively.

EXAMPLE 16

Staphylococcus aureus was suspended in a 0 85 % aqueous solution of common salt (NaCl) at a concentration of $3.0 \times 10^6$/mℓ. To 20 mℓ of the obtained suspension, was added 2.0 g of the microbial adsorption polymer obtained in Synthesis Example 12. The mixture was stirred at 150 rpm while being maintained at 37 °C. As a result, the bacterial count was lowered to $2.5 \times 10^5$/mℓ, $2.0 \times 10^3$/mℓ and $1.8 \times 10^2$/mℓ after 1, 3 and 5 hours, respectively.

EXAMPLE 17

Streptococcus faecalis was suspended in a 0.85 % aqueous solution of common salt (NaCl at a concentration of $5.0 \times 10^6$/mℓ. To 20 mℓ of the obtained suspension, was added 2.0 g of the microbial adsorption polymer obtained in Synthesis Example 13-(ii). The mixture was stirred at 150 rpm while being maintained at 37 °C. As a result, the bacterial count was lowered to $3.8 \times 10^5$/mℓ, $2.5 \times 10^3$/mℓ and $1.8 \times 10^2$/mℓ after 1, 3 and 5 hours, respectively.

EXAMPLE 18

Escherichia coli was suspended in a 0.85 % aqueous solution of common salt at a concentration of $7.8 \times 10^6$/mℓ. To 20 mℓ of the obtained suspension, was added 2.0 g of the microbial adsorption polymer obtained in Synthesis Example 14-(ii). The mixture was stirred at 150 rpm while being maintained at 37 °C. As a result, the bacterial count was lowered to $6.2 \times 10^5$/ml, $4.8 \times 10^4$/mℓ, $3.5 \times 10^3$/mℓ and $2.3 \times 10^2$/mℓ after 1, 2, 3 and 5 hours, respectively.

Example 19

Escherichia coli was suspended in a 0.85 % aqueous solution of common salt (NaCl) at a concentration of $6.0 \times 10^6$/mℓ. To 20 mℓ of the obtained suspension, was added 2.0 g of the microbial adsorption polymer obtained in Synthesis Example 15-(ii). The mixture was stirred at 150 rpm while being maintained at 37 °C. As a result, the bacterial count was lowered to $4.5 \times 10^4$/mℓ, $3.2 \times 10^3$/mℓ and $2.1 \times 10^2$/mℓ after 1, 3 and 5 hours, respectively.

## EXAMPLE 20

Streptococcus faecalis was suspended in a 0.85 % aqueous solution of common salt (NaCl) at a concentration of $3.0 \times 10^6$/mℓ. To 20 mℓ of the obtained suspension, was added 2.0 g of the microbial adsorption polymer obtained in Synthesis Example 16. The mixture was stirred at 150 rpm while being maintained at 37 °C. As a result, the bacterial count was lowered to $2.1 \times 10^5$/mℓ, $1.8 \times 10^3$/mℓ and $1.2 \times 10^2$/mℓ after 1, 3 and 5 hours, respectively.

## EXAMPLE 21

Bacteriophage $T_4$ was suspended in a 0.85 % aqueous solution of common salt (NaCl) at a concentration of $5.4 \times 10^6$/mℓ. To 20 mℓ of the obtained suspension, was added 2.0 g of the microbial adsorption polymer obtained in Synthesis Example 10-(ii). The mixture was stirred at 150 rpm while being maintained at 37 °C. As a result, the bacterial count was lowered to $3.8 \times 10^5$/mℓ, $2.3 \times 10^3$/mℓ and $1.5 \times 10^2$/mℓ after 1, 3 and 5 hours, respectively.

## EXAMPLE 22

The microbial adsorption polymer obtained in Example 15-(ii) was packed into a membrane filter holder (inner diameter: 35 cm). Then Escherichia coli suspended in a 0.85 % aqueous solution of common salt (NaCl) at a concentration of $4.0 \times 10^4$/mℓ was passed therethrough at a flow rate of 100 mℓ/min. The bacterial count of the water filtered through the microbial adsorption polymer, which was in the form of a fabric, was lowered to $2.0 \times 10^2$/mℓ.

## EXAMPLE 23

The microbial adsorption polymer obtained in Example 19 was packed into a column (inner diameter: 20 mm, length: 1000 mm). Then Staphylococcus aureus suspended in a 0.85 % aqueous solution of common salt (NaCl) at a concentration of $2.0 \times 10^6$/mℓ was passed therethrough at a flow rate of 10 mℓ min. The bacterial count showed that the eluate was completely free of the abovementioned bacterium.

## EXAMPLE 24

Evaluation of microbial adsorbability of crosslinking polymer beads:

5 g of a microbial adsorption carrier and 50 mℓ of a suspension in an activated sludge tank were introduced into a 100 mℓ Erlenmeyer flask and gently shaken at 25 °C for 2 hours. Next, the mixture was filtered, washed with a physiological saline solution, dried and weighed. Thus the amount of the adsorbed microorganisms was determined as the weight gain. Table 5 shows the results of the above test on crosslinking polymer beads (1) to (5) obtained in Synthesis Example 17 to 1, respectively.

Table 5

| Crosslinking polymer beads | Amount of adsorbed aerobic microorganism (mg/g of resin) |
|---|---|
| (1) | 107 |
| (2) | 115 |
| (3) | 130 |
| (4) | 105 |
| (5) | 40 |

As can be seen from the results in Table 5, more than 100 mg of aerobic microorganisms were adsorbed per gram of each crosslinking polymer beads utilized, except for those obtained by using polyvinyl alcohol as a dispersion stabilizer.

While the invention has been described in detail and with reference to specific examples thereof, it will be apparent to one skilled in the art that various changes and modifications can be made therein without departing from the spirit and scope thereof.

## Claims

1. A microbial sensor comprising a microbial adsorbent in which an aerobic microorganism is adsorbed, wherein said microbial sensor comprises:

a container packed with said microbial adsorbent selected from

(a) a polymer whose surface is coated with a polymer containing in the molecule thereof a pyridinium group represented by the formula (I):

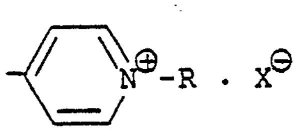

(I)

wherein R represents a benzyl group, a phenethyl group, an alkyl group having 1 to 16 carbon atoms, or a pentafluorophenylmethyl group, and X represents a halogen atom; or

(b) a weakly basic anion exchange resin;

and an electrochemical device which can determine a material formed or consumed under the effects of the microorganism, said container and said electrochemical device being separately provided in said microbial sensor.

2. The microbial sensor as claimed in claim 1, wherein said microbial adsorbent is crosslinking polymer beads having a repeating unit represented by the following formula (II) in the molecule thereof:

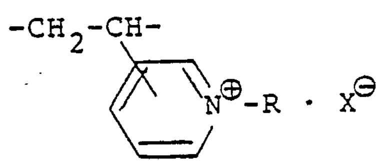

(II)

wherein R and X are as defined in above formula (I),

said crosslinking polymer beads being obtained by a method which comprises the steps:

subjecting a monomer mixture containing a vinylpyridine monomer and crosslinking monomer to suspension polymerization processing in the presence of a slightly water insoluble inorganic salt as a stabilizer for dispersion; and quaternizing a pyridyl group in the resulting polymer beads.

3. A microbial adsorbent comprising a molded product of water insoluble polymer in which the surface carries a polyvinylpyridinium polymer having a group selected from a benzyl group, a phenethyl group, an alkyl group having 1 to 16 carbon atoms, or pentafluorophenylmethyl group, as a substituent of the nitrogen atom of said polyvinylpyridinium polymer.

4. The microbial adsorbent as claimed in claim 3, wherein said microbial adsorbent is obtained by a method which comprises:
reacting a molded product of water insoluble polymer having a halomethyl group represented by the following formula (IV):
$XCH_2$- (IV)
wherein X is as defined in the above formula (I), with a vinylpyridine homopolymer or a copolymer of vinylpyridine with another monomer;
and then reacting the resulted product with an alkylating agent in which alkyl group is one selected from a benzyl group, a phenethyl group, an alkyl group having 1 to 16 carbon atoms and pentafluorophenylmethyl group.

5. The microbial sensor as claimed in claim 1, wherein said microbial adsorbent (a) is a microbial adsorbent as claimed in claim 3.

6. The microbial sensor as claimed in Claim 1, wherein said microbial adsorbent (a) is a microbial adsorbent as claimed in Claim 4.

7. The microbial sensor as claimed in Claim 1, wherein said material formed or consumed under the effects of the microorganism is dissolved oxygen.

FIG.1

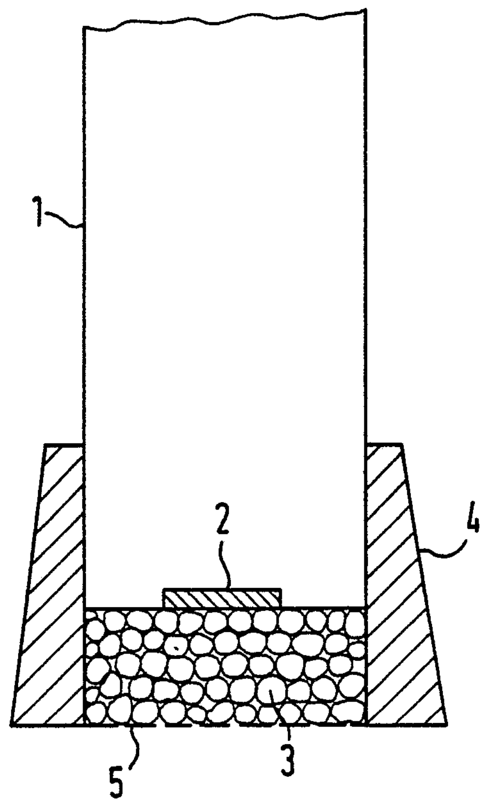


FIG.2

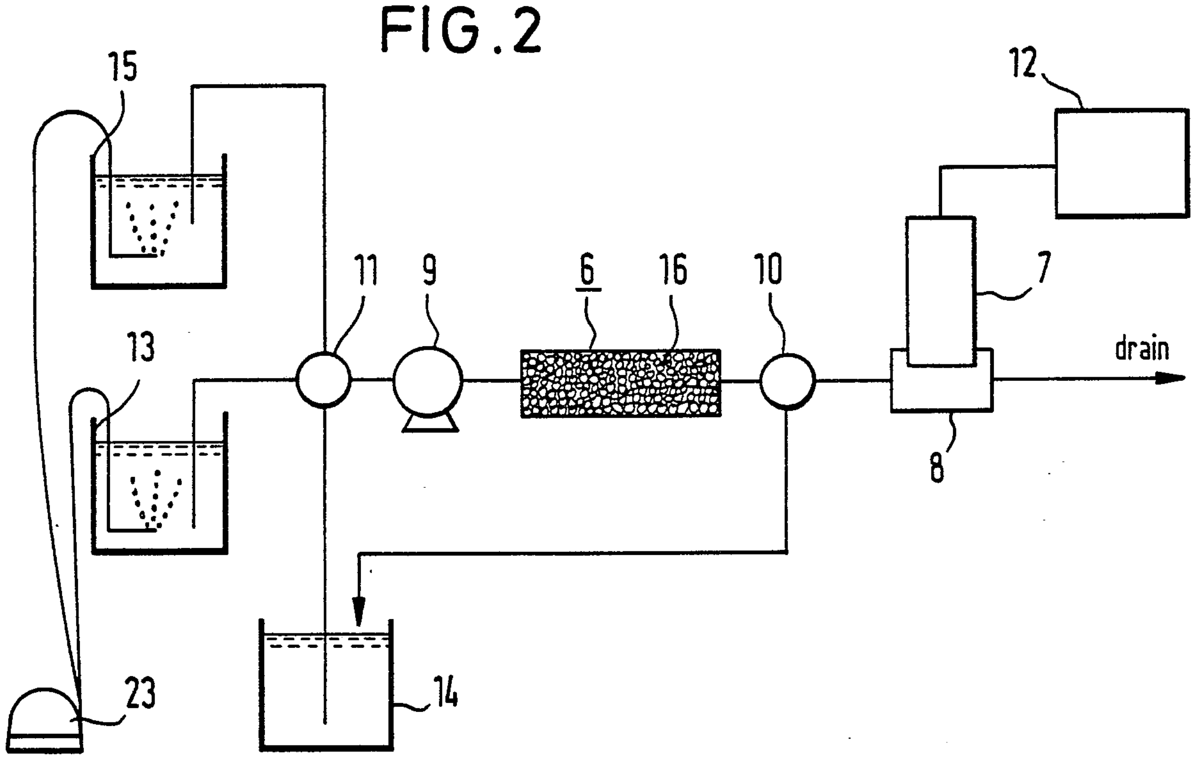

FIG.3

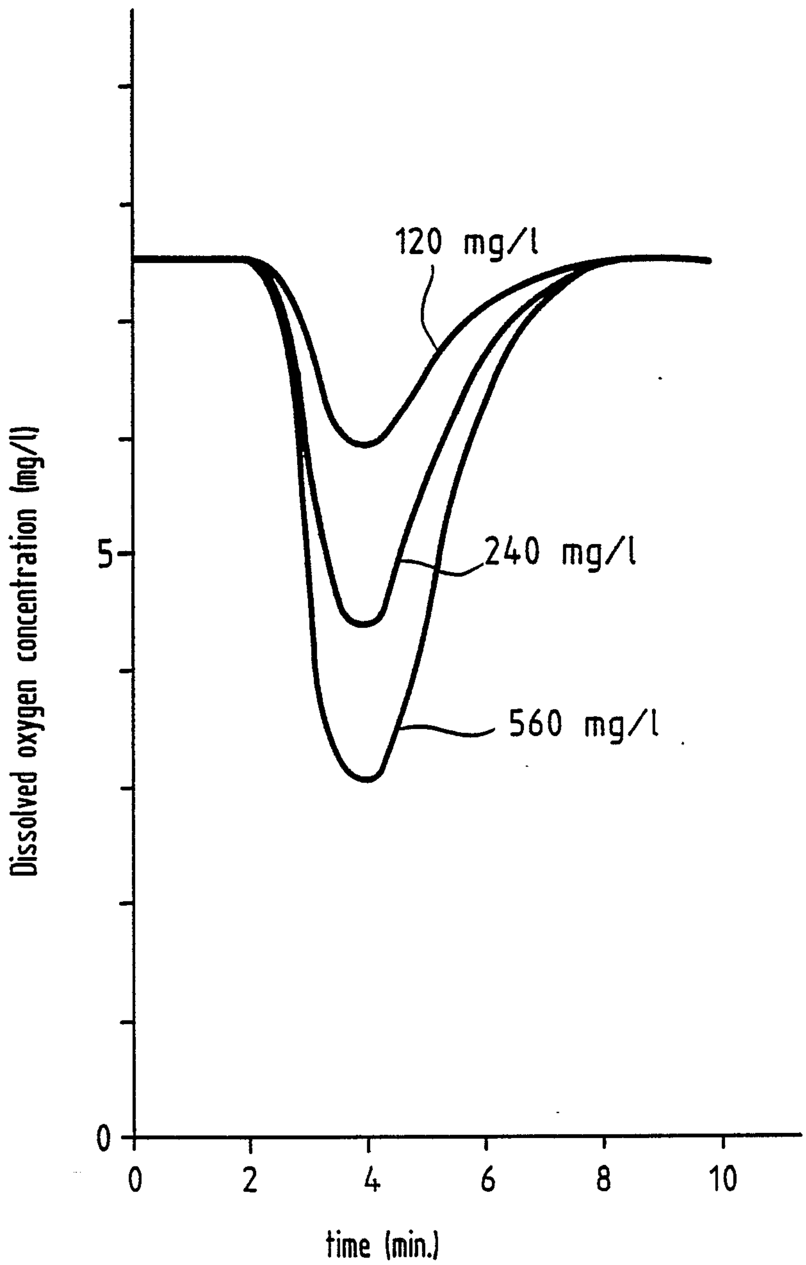
120 mg/l
240 mg/l
560 mg/l
Dissolved oxygen concentration (mg/l)
5
0
0
2
4
6
8
10
time (min.)

FIG. 4

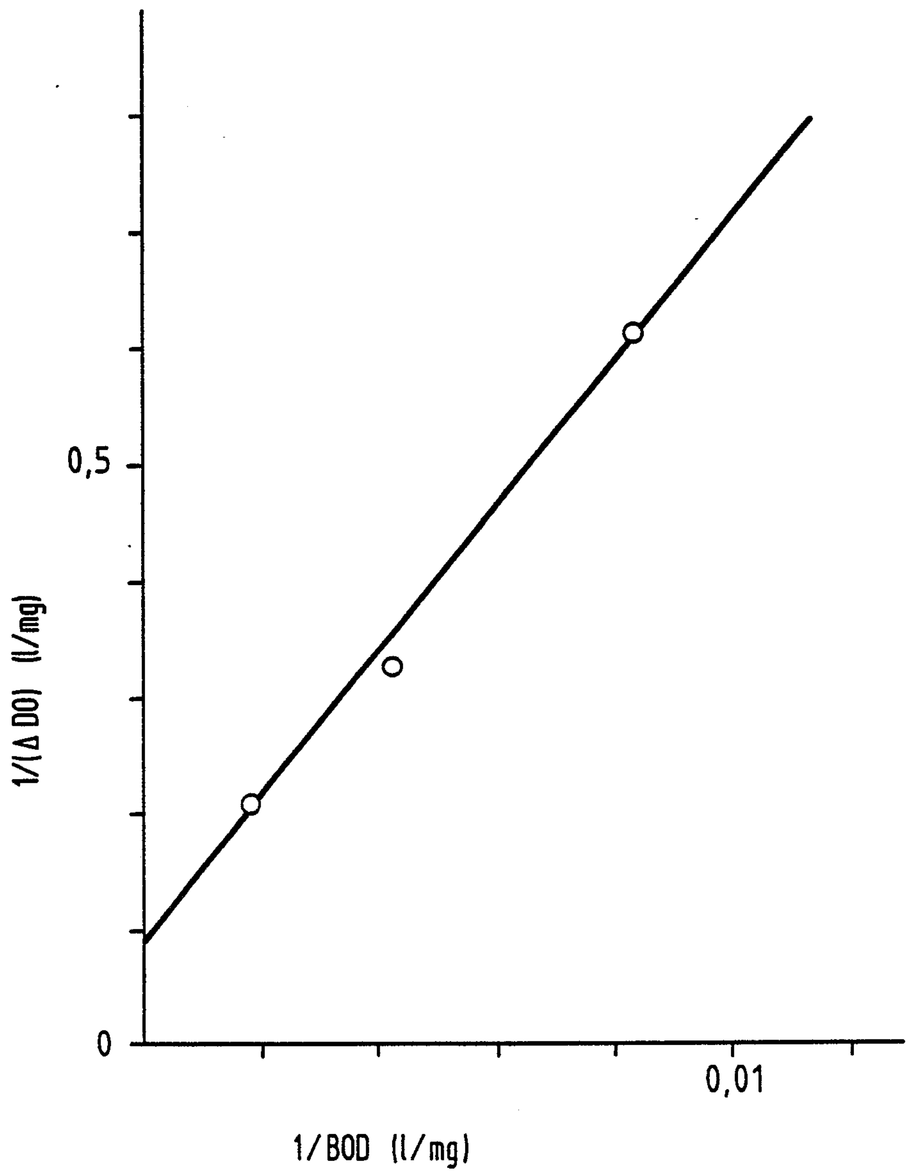

FIG. 5

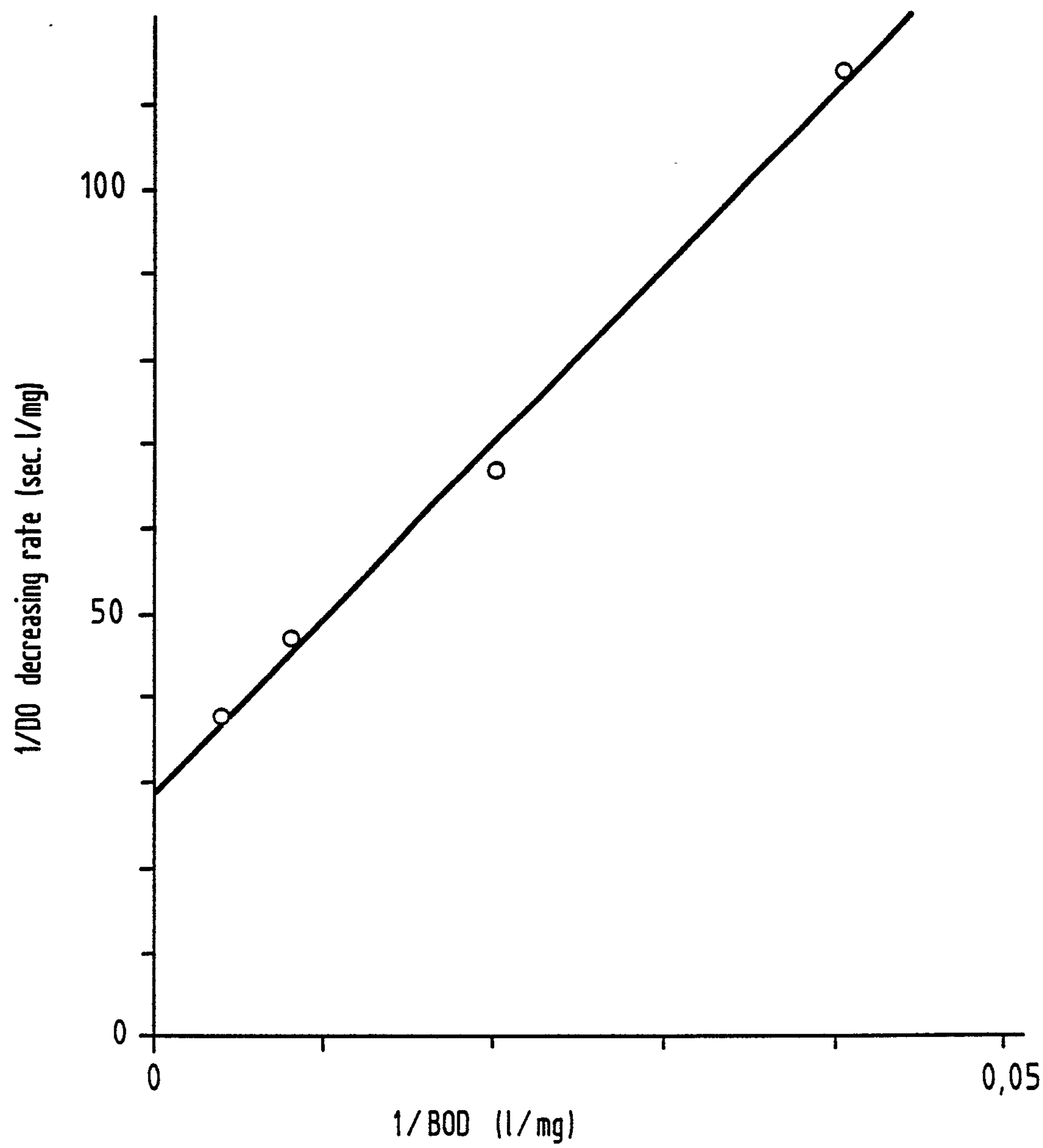

FIG. 6

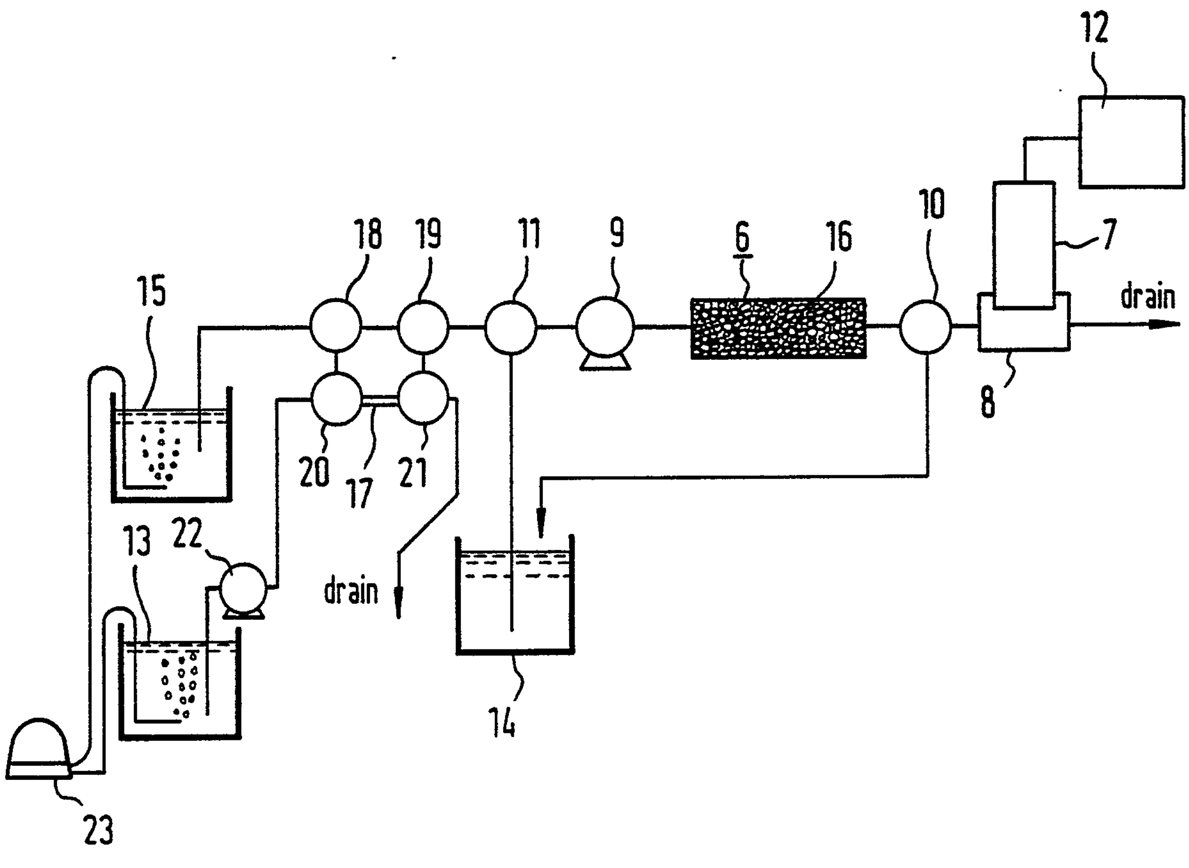

FIG. 7

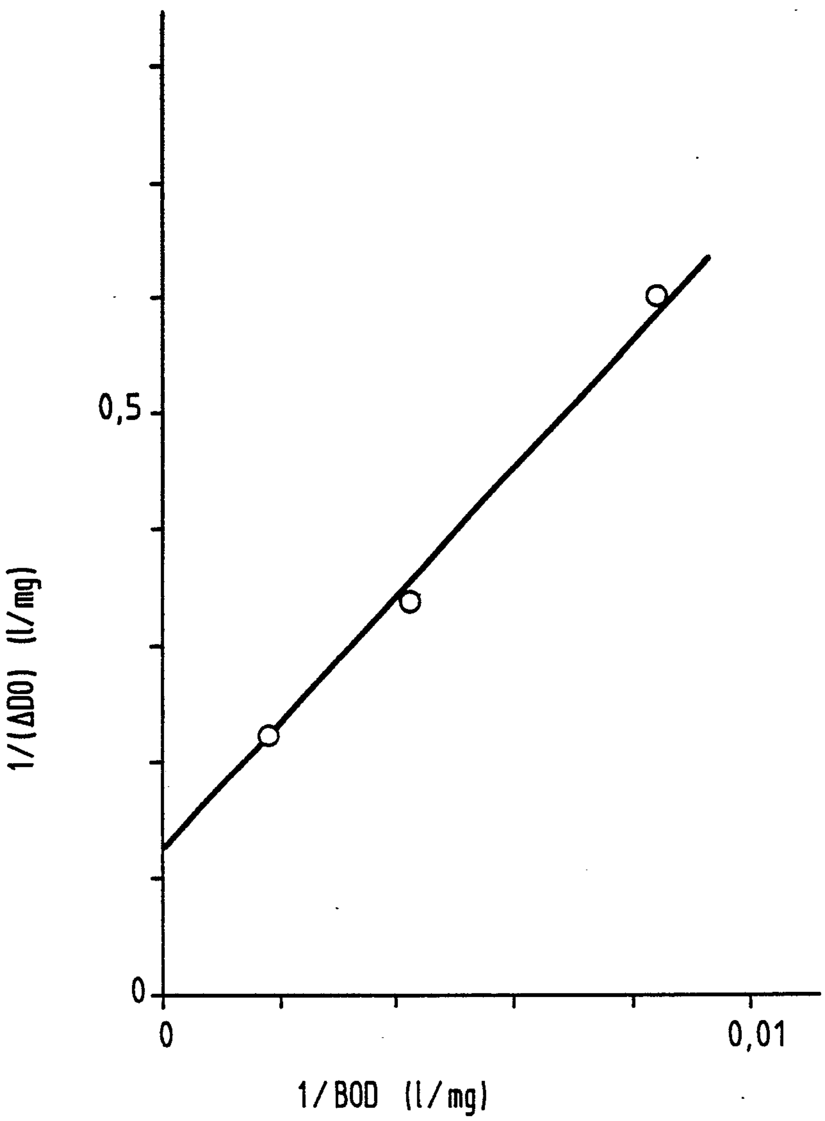

FIG.8

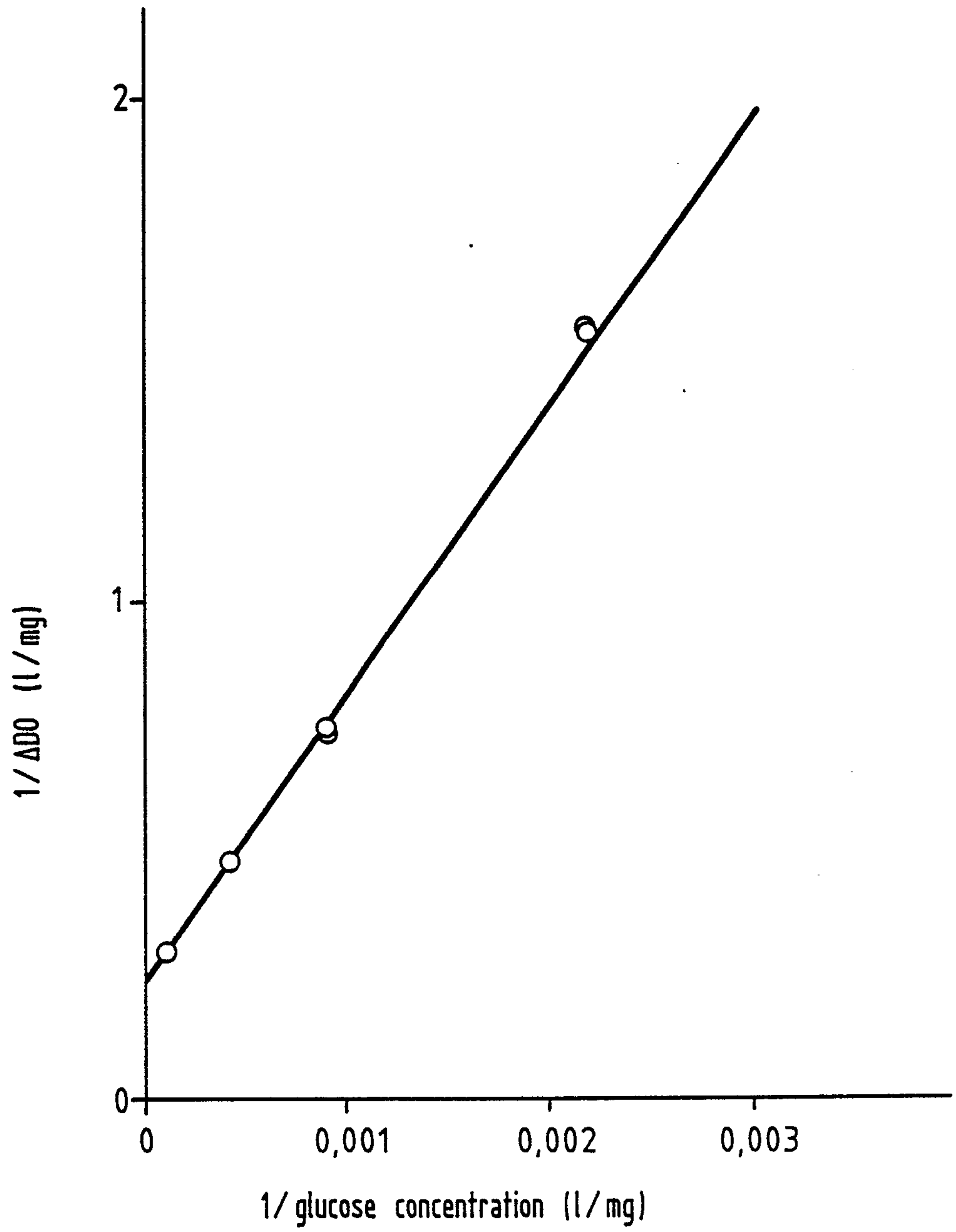

FIG.9

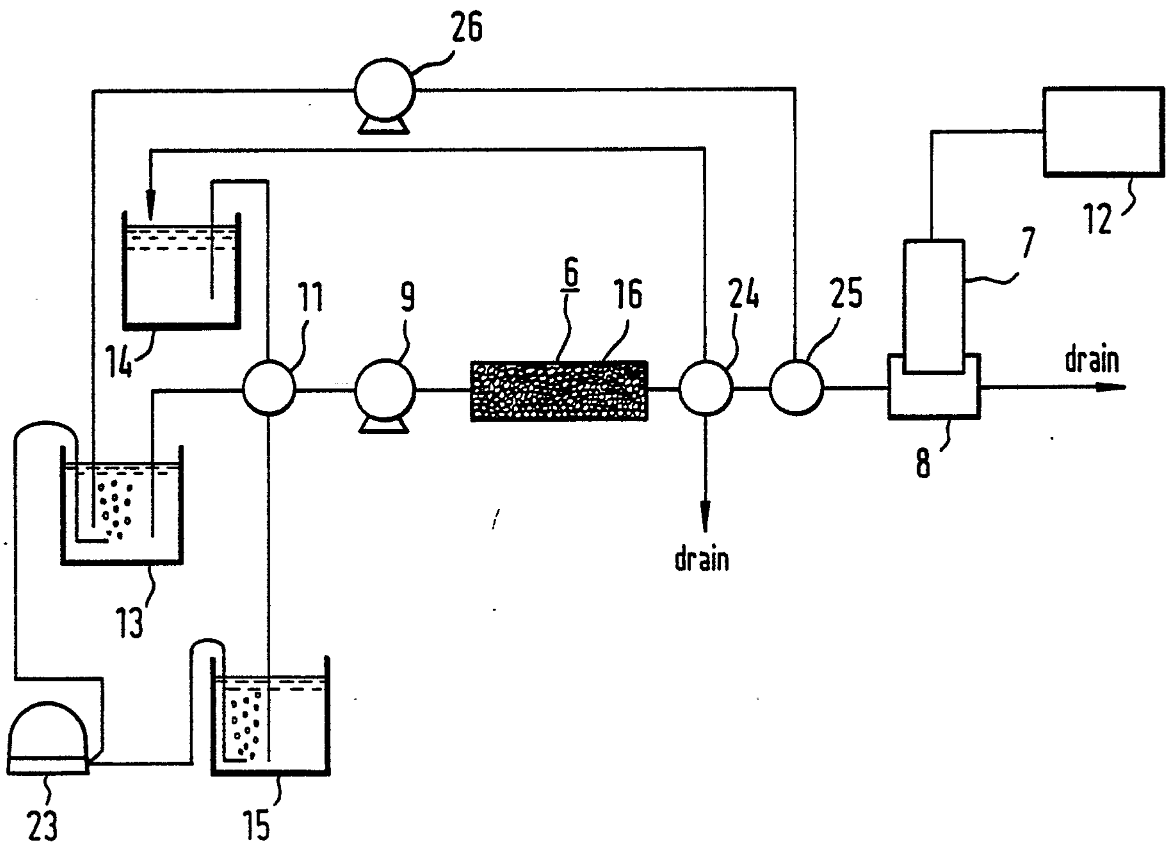

FIG.10

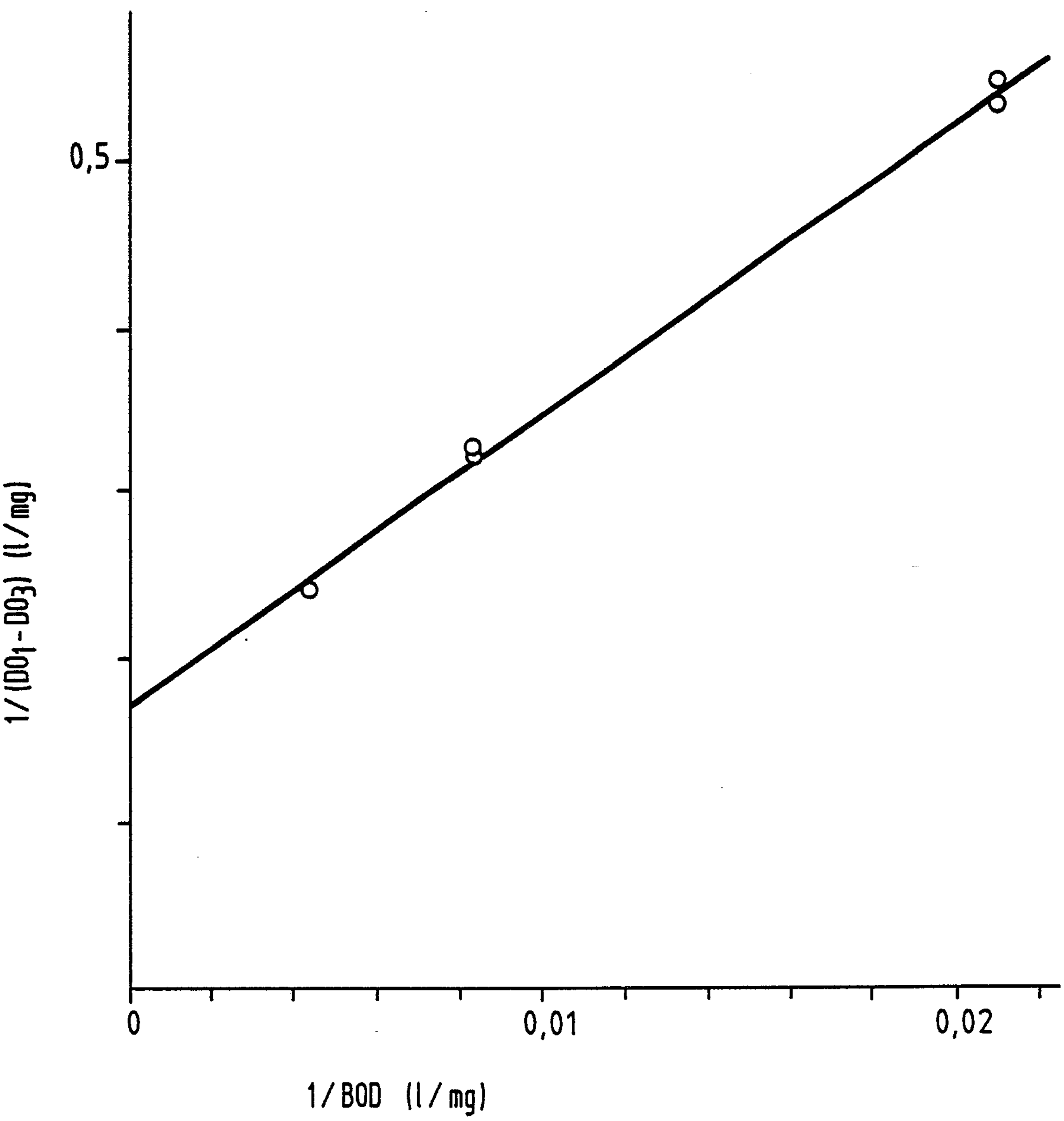

FIG.11

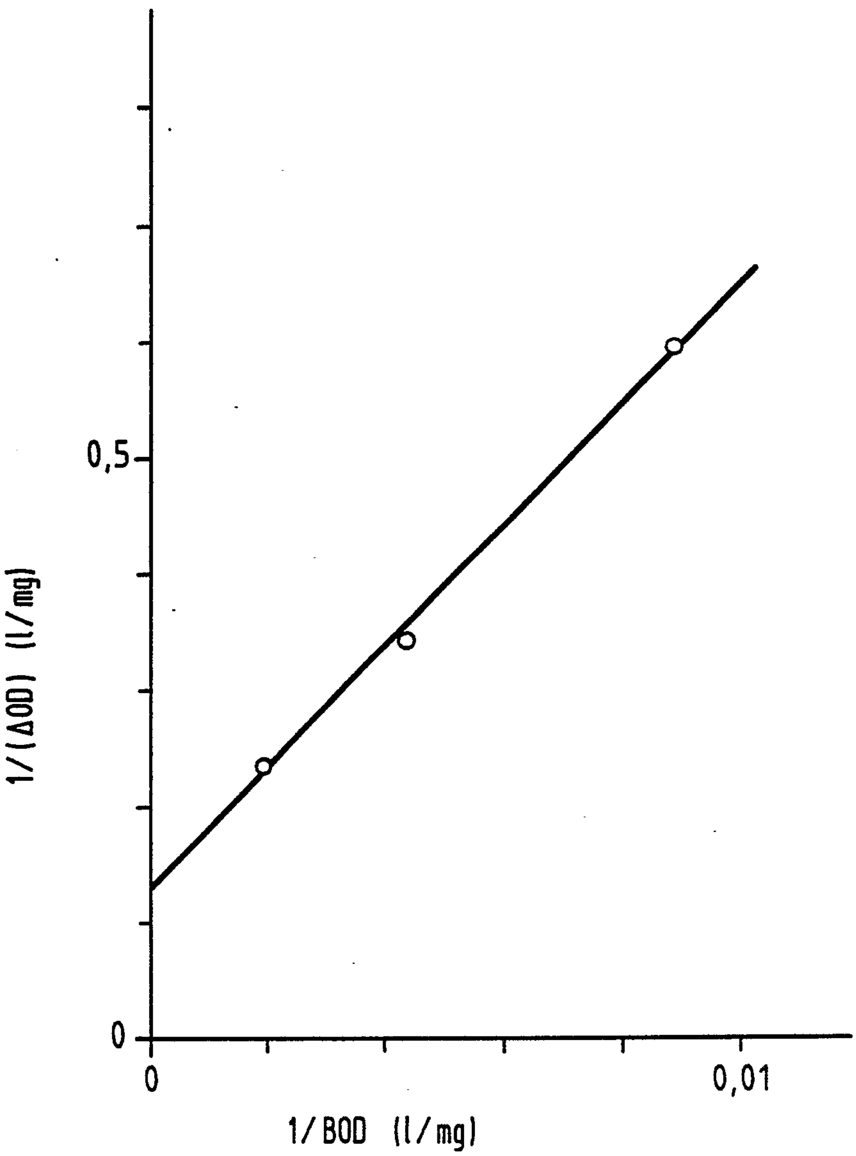

FIG.12

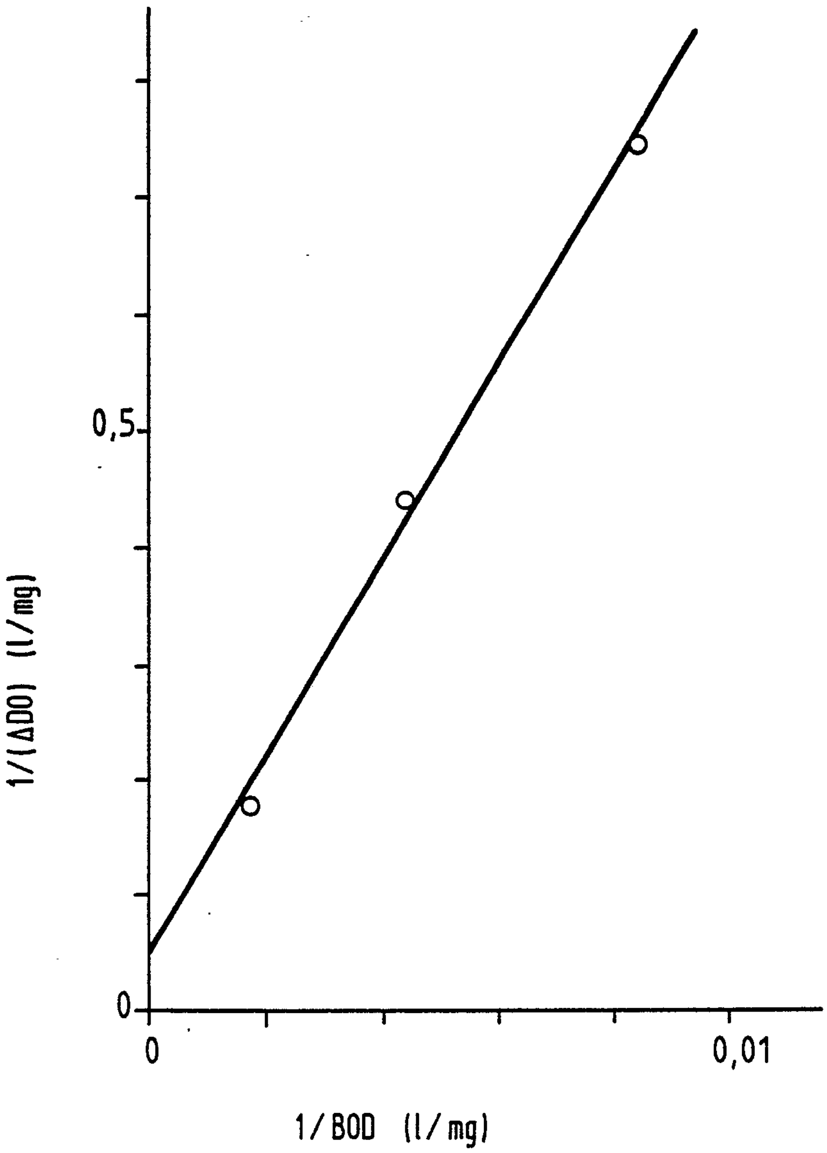

FIG . 13

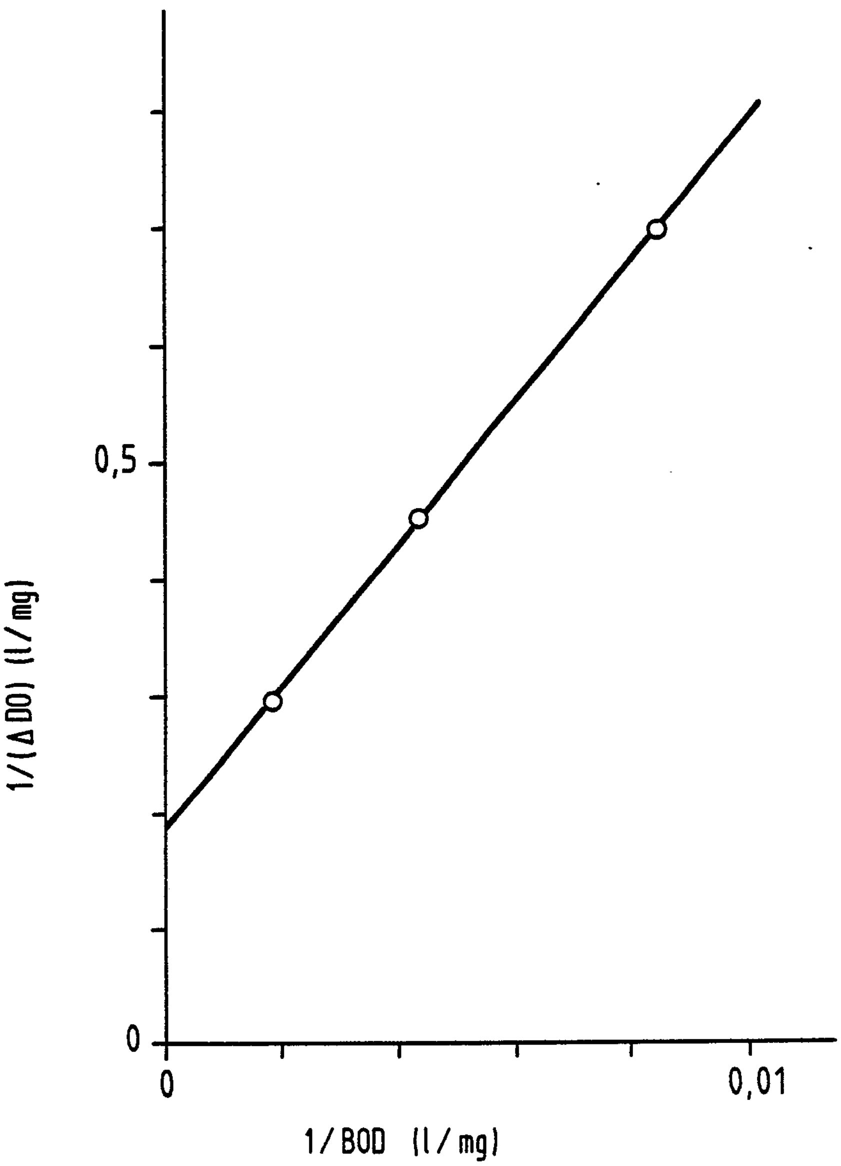

FIG.14

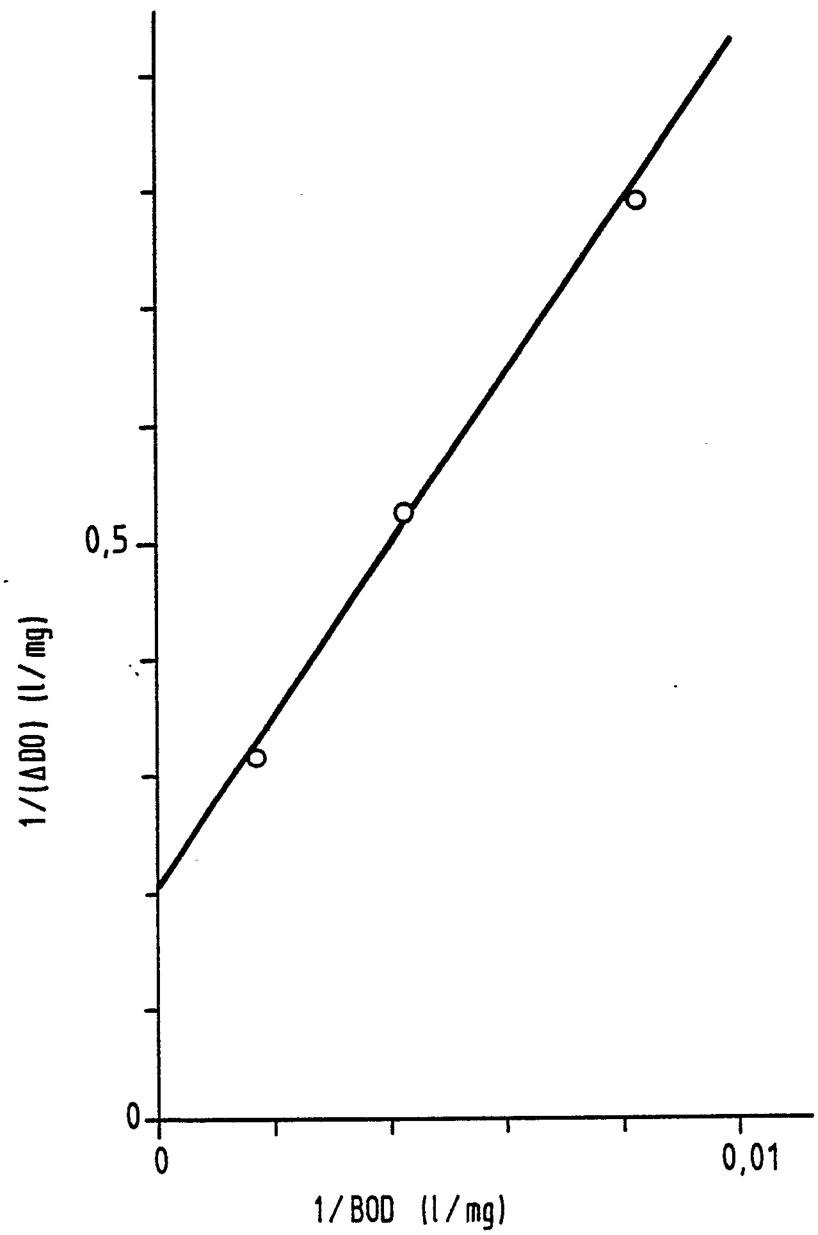

FIG.15

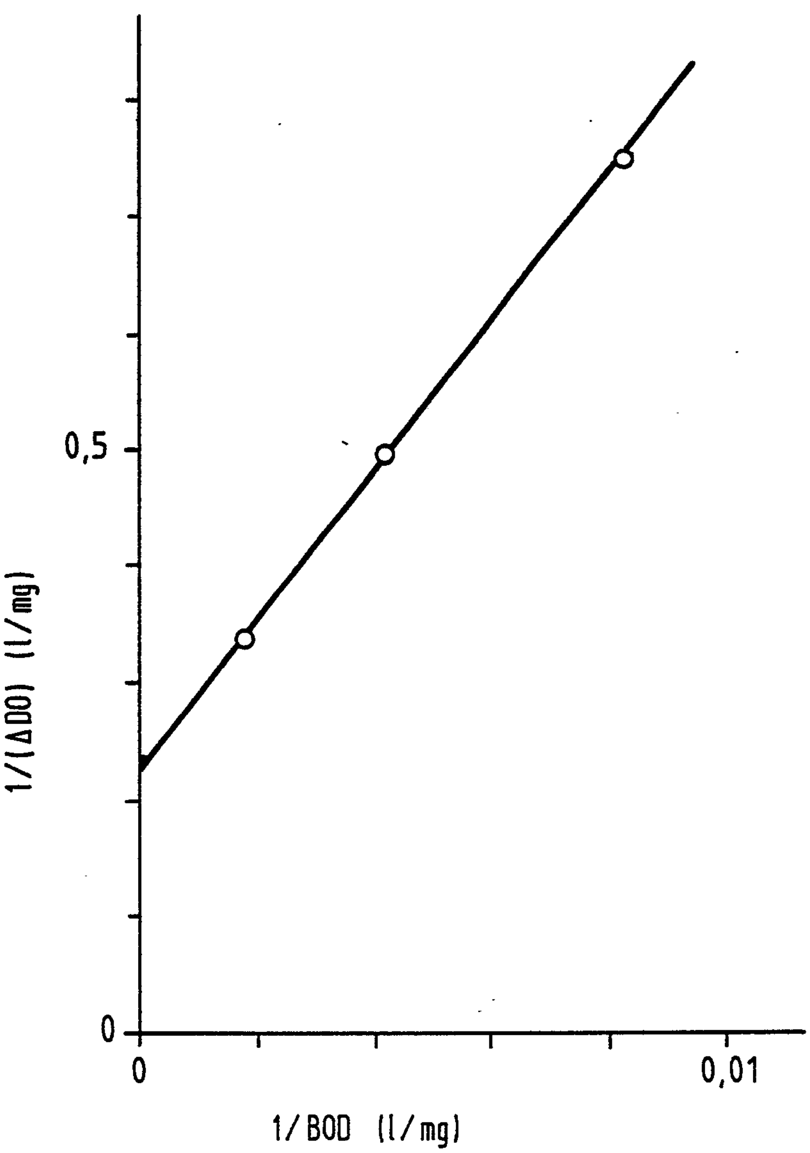

FIG. 16

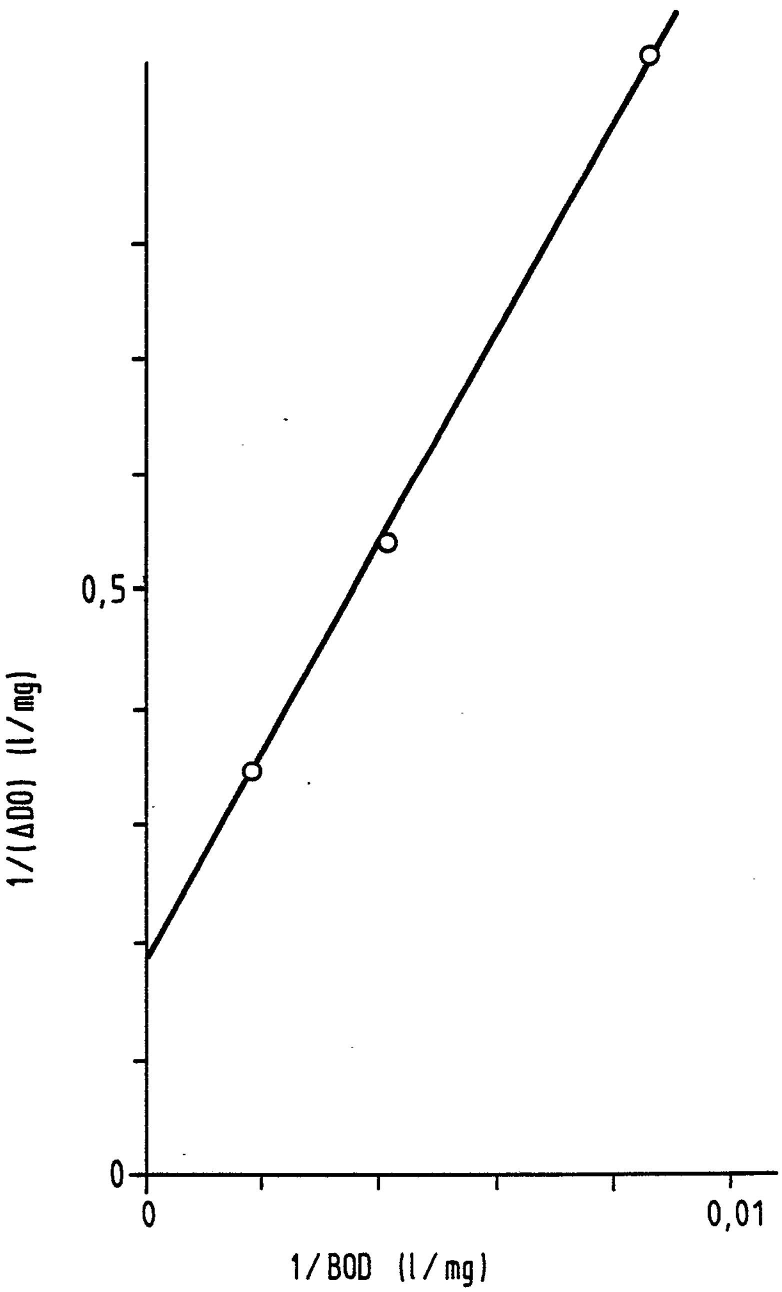

FIG.17